# EUROPÄISCHE PATENTSCHRIFT

(11) **EP 2 320 751 B1**
(45) Veröffentlichungstag und Bekanntmachung des Hinweises auf die Patenterteilung: **07.05.2014**
(21) Anmeldenummer: 09772501.4
(22) Anmeldetag: 02.07.2009
(51) Int. Cl.: A23L 1/305, A23C 9/13, A23C 9/152, A23C 19/05, A23J 3/34, C07K 5/065, C07K 5/062, A23K 1/16, A61K 38/01, A61K 38/05, C12P 21/06

(54) **MOLKENPROTEINHYDROLYSATE ENTHALTEND TRYPTOPHANHALTIGE PEPTIDE AUS ALPHA-LACTALBUMIN UND DEREN VERWENDUNG**
WHEY PROTEIN HYDROLYSATE CONTAINING TRYPTOPHAN PEPTIDE CONSISTING OF ALPHA LACTALBUMIN AND THE USE THEREOF
HYDROLYSAT DE PROTÉINE DE LACTOSÉRUM RENFERMANT UN PEPTIDE CONTENANT DU TRYPTOPHANE, FORMÉ D'ALPHALACTALBUMINE, ET LEUR UTILISATION

(30) Priorität: 02.07.2008 DE 102008032828
(43) Veröffentlichungstag der Anmeldung: 18.05.2011
(73) Patentinhaber: Technische Universität Dresden, 01069 Dresden (DE)
(72) Erfinder: HENLE, Thomas, 01309 Dresden (DE); DEUSSEN, Andreas, 01324 Dresden (DE); MARTIN, Melanie, 01069 Dresden (DE)
(74) Vertreter: Kailuweit & Uhlemann Patentanwälte
(86) Internationale Anmeldenummer: PCT/EP2009/058328
(87) Internationale Veröffentlichungsnummer: WO 2010/000801

(56) Entgegenhaltungen:
- EP-A1- 2 039 366
- WO-A1-02/19837
- WO-A1-93/24020
- WO-A1-2006/084560
- WO-A2-2007/004876
- US-A- 6 096 713
- CHRIS J VAN PLATERINK ET AL: "Application of at-line two-dimensional liquid chromatographyâ mass spectrometry for identification of small hydrophilic angiotensin I-inhibiting peptides in milk hydrolysates" ANALYTICAL AND BIOANALYTICAL CHEMISTRY, SPRINGER, BERLIN, DE, Bd. 391, Nr. 1, 9. April 2008 (2008-04-09) , Seiten 299-307, XP019621367 ISSN: 1618-2650
- DIDELOT S ET AL: "Preparation of angiotensin-I-converting enzyme inhibitory hydrolysates from unsupplemented caprine whey fermentation by various cheese microflora" INTERNATIONAL DAIRY JOURNAL, ELSEVIER APPLIED SCIENCE, BARKING, GB LNKD- DOI:10.1016/J.IDAIRYJ.2005.09.009, Bd. 16, Nr. 9, 1. September 2006 (2006-09-01), Seiten 976-983, XP024963299 ISSN: 0958-6946 [gefunden am 2006-09-01]
- PIHLANTO-LEPPALA A ET AL: "ANGIOTENSIN I-CONVERTING ENZYME INHIBITORY PROPERTIES OF WHEY PROTEIN DIGESTS: CONCENTRATION AND CHARACTERIZATION OF ACTIVE PEPTIDES" JOURNAL OF DAIRY RESEARCH, CAMBRIDGE UNIVERSITY PRESS, CAMBRIDGE, GB LNKD- DOI:10.1017/S0022029999003982, Bd. 67, Nr. 1, 1. Januar 2000 (2000-01-01) , Seiten 53-64, XP008022966 ISSN: 0022-0299
- DEPLANQUE G ET AL: "Phase II trial of the antiangiogenic agent IM862 in metastatic renal cell carcinoma" BRITISH JOURNAL OF CANCER, Bd. 91, Nr. 9, 1. November 2004 (2004-11-01), Seiten 1645-1650, XP002582576 ISSN: 0007-0920
- VAN ELSWIJK D A ET AL: "Rapid detection and identification of angiotensin-converting enzyme inhibitors by on-line liquid chromatography-biochemical detection, coupled to electrospray mass spectrometry" JOURNAL OF CHROMATOGRAPHY, ELSEVIER SCIENCE PUBLISHERS B.V, NL LNKD- DOI:10.1016/J.CHROMA.2003.08.055, Bd. 1020, Nr. 1, 5. Dezember 2003 (2003-12-05), Seiten 45-58, XP004687429 ISSN: 0021-9673
- FITZGERALD RICHARD J ET AL: "Hypotensive peptides from milk proteins" JOURNAL OF NUTRITION, Bd. 134, Nr. 4, April 2004 (2004-04), Seiten 980S-988S, XP002582577 ISSN: 0022-3166
- JAUHIAINEN T ET AL: "Milk peptides and blood pressure" JOURNAL OF NUTRITION, WISTAR INSTITUTE OF ANATOMY AND BIOLOGY, PHILADELPHIA, PA, US, Bd. 137, Nr. 3, Suppl. S, 1. März 2007 (2007-03-01), Seiten 825S-829S, XP002535671 ISSN: 0022-3166
- MARTIN MELANIE ET AL: "Identification and quantification of inhibitors for angiotensin-converting enzyme in hypoallergenic infant milk formulas" JOURNAL OF AGRICULTURAL AND FOOD CHEMISTRY, Bd. 56, Nr. 15, August 2008 (2008-08), Seiten 6333-6338, XP002603460 ISSN: 0021-8561
- SATO MINORU ET AL: "ANGIOTENSIN I-CONVERTING ENZYME INHIBITORY PEPTIDES DERIVED FROM WAKAME (UNDARIA PINNATIFIDA) AND THEIR ANTIHYPERTENSIVE EFFECT IN SPONTANEOUSLY HYPERTENSIVE RATS" JOURNAL OF AGRICULTURAL AND FOOD CHEMISTRY, AMERICAN CHEMICAL SOCIETY, US LNKD- DOI:10.1021/JF020482T, Bd. 50, Nr. 21, 14. September 2002 (2002-09-14), Seiten 6245-6252, XP009080782 ISSN: 0021-8561
- ONO S ET AL: "Isolation of peptides with angiotensin I-converting enzyme inhibitory effect derived from hydrolysate of upstream chum salmon muscle." JOURNAL OF FOOD SCIENCE, Bd. 68, Nr. 5, Juni 2003 (2003-06), Seiten 1611-1614, XP002603461 ISSN: 0022-1147
- MINORU SATO ET AL: "Production of the blood pressure lowing peptides from brown alga (Undaria pinnatifida)" JOURNAL OF OCEAN UNIVERSITY OF CHINA, Bd. 4, Nr. 3, Juli 2005 (2005-07), Seiten 209-213, XP002603462 ISSN: 1672-5182
- DATABASE WPI Week 200407 Thomson Scientific, London, GB; AN 2004-064960 XP002603481 & JP 2003 128694 A (RIKEN VITAMIN CO) 8. Mai 2003 (2003-05-08)
- DATABASE WPI Week 200633 Thomson Scientific, London, GB; AN 2006-308755 XP002603482 & JP 2006 096747 A (NICHIRO KK) 13. April 2006 (2006-04-13)
- ONO SEIGO ET AL: "Inhibition properties of dipeptides from salmon muscle hydrolysate on angiotensin I-converting enzyme" INTERNATIONAL JOURNAL OF FOOD SCIENCE & TECHNOLOGY, Bd. 41, Nr. 4, April 2006 (2006-04), Seiten 383-386, XP002603463 ISSN: 0950-5423
- KUBA M ET AL: "Production of angiotensin I-converting enzyme inhibitory peptides from soybean protein with Monascus purpureus acid proteinase" PROCESS BIOCHEMISTRY, ELSEVIER, NL LNKD- DOI:10.1016/J.PROCBIO.2004.08.010, Bd. 40, Nr. 6, 1. Mai 2005 (2005-05-01), Seiten 2191-2196, XP025306538 ISSN: 1359-5113 [gefunden am 2005-05-01]
- KUBA M ET AL: "Angiotensin I-converting enzyme inhibitory peptides isolated from tofuyo fermented soybean food" BIOSCIENCE BIOTECHNOLOGY BIOCHEMISTRY, JAPAN SOCIETY FOR BIOSCIENCE, BIOTECHNOLOGY, AND AGROCHEMISTRY, TOKYO, JAPAN LNKD- DOI:10.1271/BBB.67.1278, Bd. 67, Nr. 6, 1. Januar 2003 (2003-01-01) , Seiten 1278-1283, XP003007052 ISSN: 0916-8451

### SEQUENCE LISTING

<110> Technische Universität Dresden
<120> Molkenproteinhydrolysate enthaltend tryptophanhaltige Peptide aus alpha-Lactalbumin und deren Verwendung
<130> 0017P0114DEWO
<160> 32
<170> PatentIn version 3.5
<210> 1
   <211> 123
   <212> PRT
   <213> Bos taurus
<400> 1
   Glu Gln Leu Thr Lys Cys Glu Val Phe Arg Glu Leu Lys Asp Leu Lys
   1               5                   10                  15
   Gly Tyr Gly Gly Val Ser Leu Pro Glu Trp Val Cys Thr Thr Phe His
               20                  25                  30
   Thr Ser Gly Tyr Asp Thr Gln Ala Ile Val Gln Asn Asn Asp Ser Thr
           35                  40                  45
   Glu Tyr Gly Leu Phe Gln Ile Asn Asn Lys Ile Trp Cys Lys Asp Asp
       50                  55                  60
   Gln Asn Pro His Ser Ser Asn Ile Cys Asn Ile Ser Cys Asp Lys Phe
   65                  70                  75                  80
   Leu Asp Asp Asp Leu Thr Asp Asp Ile Met Cys Val Lys Lys Ile Leu
                   85                  90                  95
   Asp Lys Val Gly Ile Asn Tyr Trp Leu Ala His Lys Ala Leu Cys Ser
               100                 105                 110
   Glu Lys Leu Asp Gln Trp Leu Cys Glu Lys Leu
           115                 120
<210> 2
   <211> 2
   <212> PRT
   <213> Artificial Sequence
<220>
   <223> Peptid
<400> 2
   Glu Trp
   1
<210> 3
   <211> 2
   <212> PRT
   <213> Artificial Sequence
<220>
   <223> Peptid
<400> 3
   Trp Val
   1
<210> 4
   <211> 2
   <212> PRT
   <213> Artificial Sequence
<220>
   <223> Peptid
<400> 4
   Ile Trp
   1
<210> 5
   <211> 2
   <212> PRT
   <213> Artificial Sequence
<220>
   <223> Peptid
<400> 5
   Trp Cys
   1
<210> 6
   <211> 2
   <212> PRT
   <213> Artificial Sequence
<220>
   <223> Peptid
<400> 6
   Tyr Trp
   1
<210> 7
   <211> 2
   <212> PRT
   <213> Artificial Sequence
<220>
   <223> Peptid
<400> 7
   Trp Leu
   1
<210> 8
   <211> 2
   <212> PRT
   <213> Artificial Sequence
<220>
   <223> Peptid
<400> 8
   Gln Trp
   1
<210> 9
   <211> 3
   <212> PRT
   <213> Artificial Sequence
<220>
   <223> Peptid
<400> 9 <210> 10
   <211> 3
   <212> PRT
   <213> Artificial Sequence
<220>
   <223> Peptid
<400> 10
<210> 11
   <211> 3
   <212> PRT
   <213> Artificial Sequence
<220>
   <223> Peptid
<400> 11
   Lys Ile Trp
   1
<210> 12
   <211> 3
   <212> PRT
   <213> Artificial Sequence
<220>
   <223> Peptid
<400> 12
   Trp Cys Lys
   1
<210> 13
   <211> 3
   <212> PRT
   <213> Artificial Sequence
<220>
   <223> Peptid
<400> 13
   Asn Tyr Trp
   1
<210> 14
   <211> 3
   <212> PRT
   <213> Artificial Sequence
<220>
   <223> Peptid
<400> 14 <210> 15
<211> 3
   <212> PRT
   <213> Artificial Sequence
<220>
   <223> Peptid
<400> 15
<210> 16
   <211> 3
   <212> PRT
   <213> Artificial Sequence
<220>
   <223> Peptid
<400> 16
   Trp Leu Cys
   1
<210> 17
   <211> 4
   <212> PRT
   <213> Artificial Sequence
<220>
   <223> Peptid
<400> 17
   Leu Pro Glu Trp
   1
<210> 18
   <211> 4
   <212> PRT
   <213> Artificial Sequence
<220>
   <223> Peptid
<400> 18
   Trp Val Cys Thr
   1
<210> 19
   <211> 4
   <212> PRT
   <213> Artificial Sequence
<220>
   <223> Peptid
<400> 19
   Asn Lys Ile Trp
   1
<210> 20
   <211> 4
   <212> PRT
   <213> Artificial Sequence
<220>
   <223> Peptid
<400> 20
   Trp Cys Lys Asp
   1
<210> 21
   <211> 4
   <212> PRT
   <213> Artificial Sequence
<220>
   <223> Peptid
<400> 21
   Ile Asn Tyr Trp
   1
<210> 22
   <211> 4
   <212> PRT
   <213> Artificial Sequence
<220>
   <223> Peptid
<400> 22
   Trp Leu Ala His
   1
<210> 23
   <211> 4
   <212> PRT
   <213> Artificial Sequence
<220>
   <223> Peptid
<400> 23
   Leu Asp Gln Trp
   1
<210> 24
   <211> 4
   <212> PRT
   <213> Artificial Sequence
<220>
   <223> Peptid
<400> 24
   Trp Leu Cys Glu
   1
<210> 25
   <211> 5
   <212> PRT
   <213> Artificial Sequence
<220>
   <223> Peptid
<400> 25
   Ser Leu Pro Glu Trp
   1 5
<210> 26
   <211> 5
   <212> PRT
   <213> Artificial Sequence
<220>
   <223> Peptid
<400> 26
   Trp Val Cys Thr Thr
   1 5
<210> 27
   <211> 5
   <212> PRT
   <213> Artificial Sequence
<220>
   <223> Peptid
<400> 27
   Asn Asn Lys Ile Trp
   1 5
<210> 28
   <211> 5
   <212> PRT
   <213> Artificial Sequence
<220>
   <223> Peptid
<400> 28
   Trp Cys Lys Asp Asp
   1 5
<210> 29
   <211> 5
   <212> PRT
   <213> Artificial Sequence
<220>
   <223> Peptid
<400> 29
   Gly Ile Asn Tyr Trp
   1 5
<210> 30
   <211> 5
   <212> PRT
   <213> Artificial Sequence
<220>
   <223> Peptid
<400> 30
   Trp Leu Ala His Lys
   1 5
<210> 31
   <211> 5
   <212> PRT
   <213> Artificial Sequence
<220>
   <223> Peptid
<400> 31
   Lys Leu Asp Gln Trp
   1 5
<210> 32
   <211> 5
   <212> PRT
   <213> Artificial Sequence
<220>
   <223> Peptid
<400> 32
   Trp Leu Cys Glu Lys
   1 5

## Beschreibung

Die Erfindung betrifft Molkenproteinhydrolysate, insbesondere Hydrolysate aus mit α-Lactalbumin angereichertem Molkenprotein und aus α-Lactalbumin, und deren Verwendung für die Herstellung von Arzneimitteln, blutdrucksenkenden Mitteln, Nahrungsergänzungsmitteln, Nahrungsprodukten und Futtermitteln, und die auf diese Weise hergestellten Arzneimittel, blutdrucksenkenden Mittel, Nahrungsergänzungsmittel, Nahrungsprodukte und Futtermittel.

Die periphere arterielle Hypertonie (Bluthochdruck) ist eine Volkskrankheit, die in den westlichen Industrienationen ab dem 50. Lebensjahr jeden zweiten Erwachsenen betrifft (systolischer Blutdruck über 140 mm Hg). Bluthochdruck führt zu einer Reihe von Folgeerkrankungen, so zu degenerativen Veränderungen der Gefäße (Atherosklerose) mit arterieller Verschlusskrankheit (koronare Herzkrankheit, Zerebralinsuffizienz, peripher arterielle Verschlusskrankheit), Herzhypertrophie, Herzinfarkt und Herzinsuffizienz sowie Schlaganfall. Das Risiko dieser Komplikationen steigt nahezu exponentiell mit der Erhöhung des systolischen und diastolischen Blutdrucks an, wobei kein Schwellenwert existiert. m Vergleich zu einem systolischen Blutdruck von 110 mm Hg ist das Risiko, an einem Herzinfarkt zu versterben, bei einem Druck von 145 mm Hg verdoppelt und bei 160 mm Hg verdreifacht. Integraler Bestandteil der modernen Bluthochdrucktherapie ist die Hemmung des sogenannten Renin-Angiotensin-Aldosteron-Systems durch Hemmung der Aktivität von Angiotensin Converting Enzyme (ACE), einem Verfahren, dessen Wirksamkeit seit Anfang der 1980er Jahre durch den Einsatz von sogenannten ACE-Hemmern umfassend dokumentiert ist. Schätzungen zufolge werden in Deutschland heute etwa 20 % der Bevölkerung bzw. jeder zweite im Alter über 55 Jahre mit Arzneimitteln gegen zu hohen Blutdruck behandelt. Die durch das Gesundheitssystem zu tragenden Kosten betrugen in der BRD im Jahre 2005 etwa 1,5 Milliarden Euro, wovon etwa die Hälfte auf die Verschreibung von ACE-Hemmern entfällt (Schwabe U. und Pfaffrath E. Arzneiverordnungs-Report 2006. Springer, Berlin, 2006). Vor diesem Hintergrund sind neuartige Präventionswege notwendig, um eine weitere Reduktion der kardiovaskulären Todesfälle infolge Bluthochdrucks erreichen zu können. Dies könnte durch das Einbringen wirksamer Inhaltsstoffe in die tägliche Nahrung erfolgen, um so die altersabhängige Entwicklung des arteriellen Blutdrucks günstig zu beeinflussen.

Bluthochdruck ist auch in der Tiermedizin ein in den letzten Jahren herausragendes Problem geworden, welches direkte Behandlungskosten für Tierbesitzer nach sich zieht. Schätzungen zufolge gibt es in Deutschland ca. 23 Millionen Haustiere, davon etwa 5,3 Mio. Hunde und 7,5 Mio. Katzen (Statistisches Jahrbuch für Deutschland, 2006). Wegen der zunehmenden Lebensdauer dieser Haustiere wird der Bluthochdruck auch hier zunehmend zu einem medizinischen Problem.

Neben der individuell-sozialen Bedeutung der Haustierhaltung kommt dieser auch eine erhebliche wirtschaftliche Bedeutung zu. So betrug der Umsatz an Tiernahrung in Deutschland im Jahre 2008 etwa 2,6 Milliarden Euro, wovon mit weit über 90 % der größte Teil auf Hunde- und Katzennahrung entfiel (Quelle: Industrieverband Heimtierbedarf IVH e.V., "The German PET Market - Structure and Sales Data" http://www.ivh-online.de/fileadmin/user_upload/German_Pet_Market_2008_A4.pdf). Studien deuten darauf hin, dass etwa 61 % der Katzen und bis zu 93 % der Hunde aufgrund ihrer "optimalen" Haltungsbedingungen und damit verbundenen hohen Lebenserwartung im Laufe ihres Lebens eine Hypertonie entwickeln (Aciemo M. J., Labato M. A. (2005) Hypertension in renal disease Clin. Tech. Small Anim. Pract. 20:23-30). Bluthochdruck tritt dabei oft im Zusammenhang mit altersbedingten Nierenerkrankungen oder anderen metabolischen Störungen auf, was zu Nierenversagen, Erblindungen und neurologischen Komplikationen führen kann (Brown S.A., Henik R.A. (1998) Diagnosis and treatment of systemic hypertension. Vet. Clin. North Am. Small Anim. Pract. 28:1481-94).

Inzwischen sind mehrere Peptide bekannt, die in der Struktur von Lebensmittelproteinen, im speziellen in Milchproteinen, vorliegen und ACE *in vitro* inhibieren können (Saito T. Antihypertensive peptides derived from bovine casein and whey proteins. Adv. Exp. Med. Biol. 2008, 606, 295-317). Die bekanntesten sind dabei die beiden Tripeptide Val-Pro-Pro und Ile-Pro-Pro, die bei der Milchfermentation mit speziellen Mikroorganismen (*Lactobacillus helveticus LBK-16H*, *Aspergillus oryzae*) durch proteolytischen Abbau aus Casein gebildet werden (Nakamura Y. et al. Purification and characterization of angiotensin I-converting enzyme inhibitors from sour milk. J. Dairy Sci. 1995, 78, 777-783). Diese auch als "Casokinine" oder "Lactotripeptide" bekannten Peptide können entweder endogen in Sauermilchprodukten gebildet oder nach vorhergehender Anreicherung bestimmten Lebensmitteln zugesetzt werden. Entsprechende funktionelle Milchprodukte zur Blutdrucksenkung sind mittlerweile in Japan ("Calpis") und mehreren europäischen Ländern ("Evolus" in Finnland, Portugal, Schweiz) kommerziell erhältlich.

Klinische Studien deuten darauf hin, dass durch die Aufnahme von Sauermilchprodukten, die Val-Pro-Pro und Ile-Pro-Pro enthalten, eine signifikante Absenkung des Blutdruckes bei Hypertonikern erreicht werden kann (Hata Y. et al. A placebo-controlled study of the effect of a sour milk on blood pressure in hypertensive subjects. Am. J. Clin. Nutr. 1996, 64, 767-771, Mizushima S. et al. Randomized controlled trial of sour milk on blood pressure in borderline hypertensive men. Am. J. Hypertens. 2004, 17, 701-706, Seppo L. et al. A fermented milk high in bioactive peptides has a blood pressure-lowering effect in hypertensive subjects. Am. J. Clin. Nutr. 2003, 77, 326-330, Mizuno S. et al. Antihypertensive effect of casein hydrolysate in a placebo-controlled study in subjects with high blood pressure and mild hypertension. Brit. J. Nutr. 2005, 94, 84-91), obwohl Studien aus jüngster Zeit widersprüchlich sind (Lee Y.M. et al. Effect of a milk drink supplemented with whey peptides on blood pressure in patients with mild hypertension. Eur. J. Nutr. 2007, 46, 21-27, Engberink M.F. et al. Lactotripeptides show no effect on human blood pressure: results from a double-blind randomized controlled trial. Hypertension 2008, 51, 399-405).

Aufgrund der oben genannten Gründe bietet es sich außerdem an, das Prinzip der Blutdrucksenkung durch ACE-inhibierende Peptide auch bei Futtermitteln für Tiere anzuwenden. Dabei wäre ein Einsatz grundsätzlich für alle Produkte denkbar (Anreicherung komplexer Feucht- und Trockenfuttermittel, spezielle "Snacks" usw.). Diese Futtermittel wären ebenso frei verkäuflich und könnten von den Tierhaltern zu wesentlich geringeren Kosten als die durch einen Tierarzt verschriebenen Medikamente angewendet werden.

Bisherige Applikationen zum Einsatz der oben genannten Peptide zeichnen sich durch folgende Nachteile aus: So nutzen sie relativ ineffiziente, d. h. relativ schwache ACE-Inhibitoren. Dadurch müssen für eine biologische Wirkung relativ hohe Konzentrationen an Peptiden im betreffenden Lebensmittel (z. B. fermentierte Milchprodukte) eingestellt werden, was sowohl aus Kostengründen wie auch aus sensorischen Aspekten ungünstig ist, denn bei der Hydrolyse von Nahrungsproteinen entstehen Peptide, die einen bitteren und unangenehmen Geschmack haben, was einen Einsatz in Lebensmitteln schwierig macht.

EP 1 087 668 B1 offenbart ein Verfahren zur Herstellung von hydrolysierten Molkenproteinprodukten, enthaltend bioaktive Peptide, die frei von bitteren Aromen sind. Dazu wird das Molkenprotein-haltige Substrat enzymatisch hydrolysiert, bis ein Hydrolysegrad von maximal 10 % erreicht ist. Ein Hydrolysegrad von 10 % entspricht einer durchschnittlichen Spaltung von 10 % der Peptidbindungen in dem hydrolysierten Protein. Es wird auch eine Reihe von in den erfindungsgemäßen Produkten enthaltenen bioaktiven Peptiden offenbart, die zwischen 2 und 19 Aminosäuren lang sind und aus den Primärsequenzen von Protease-Pepton, ß-Lactoglobulin, Glycomacropeptid und ß-Casein freigesetzt wurden.

WO 0.1/85984 offenbart ein Verfahren zur Herstellung einer Zusammensetzung mit ACE-hemmender Wirkung. Dazu wird ein Molkenproteinisolat proteolytisch verdaut. Die ACE-hemmende Wirkung wird auf keine spezifischen Peptide zurückgeführt.

WO 2006/025731 offenbart die enzymatische Herstellung eines Proteinhydrolysats mit ACE-hemmender Wirkung durch einen Verdau eines ß-Lactoglobulin-haltigen Substrats in zwei Schritten. Im ersten Schritt wird das ß-Lactoglobulin-haltige Substrat mit einer Breitband-Endoprotease, bevorzugt Alcalase, verdaut, im zweiten Schritt geschieht der Verdau durch eine Prolin-spezifische Endoprotease. Die Erfindung offenbart unter anderem auch die Ausfällung von anderen Molkenproteinen wie α-Lactalbumin bei der Verwendung von Molkenprotein als Substrat, um den Anteil an ß-Lactoglobulin im Ausgangsmaterial zu steigern.

EP 1 226 267 B1 offenbart ein Verfahren zur Herstellung eines Produktes, enthaltend antihypertensiv wirkende Peptide, durch die Fermentierung eines Casein-haltigen Ausgangsmaterials mit einem Milchsäurebakterium. Anschließend wird das Peptidenthaltende Fermentationsprodukt durch Nanofiltration und anschließende Rückgewinnung des Retentats gewonnen.

Sato *et al.* beschreiben verschiedene Peptide mit ACE-inhibitorischer Wirkung, darunter auch Ile-Trp, die durch proteolytischen Verdau von Braunalgen mit der Protease S Amano erhalten wurden (Sato M. et al. Angiotensin I-converting enzyme inhibitory peptides derived from wakame (Undaria pinnatifida) and their antihypertensive effect in spontaneously hypertensive rats. J. Agric. Food Chem. 2002; 50(21) 6245-52).

JP 2006096747 offenbart die Herstellung von bioaktiven Peptiden mit ACE-hemmender Wirkung, unter anderem auch Ile-Trp, aus Muskelfleisch oder Leber von Lachsen durch Proteaseverdau.

Kuba *et al.* beschreiben das ACE-hemmende Dipeptid Trp-Leu in traditionellen japanischen Lebensmitteln, die durch die Fermentation von Sojaprodukten hergestellt werden (Kuba et al. Angiotensin I-Converting Enzyme Inhibitory Peptides Isolated from Tufuyo Fermented Soybean Food, Biosci. Biotechnol. Biochem. 2003, 67 (6), 1278-1283).

Das Patent WO 2004/047566 offenbart Nahrungszusammensetzungen für Patienten mit Leberschäden sowie nach Operationen, Infektionen oder Verbrühungen, die - neben einem an Fettsäuren reichen Öl sowie Milch- oder Soja-Lecithin als Lipidbestandteil - als Proteinbestandteil Milchproteinhydrolysat und Protein, das aus fermentierten Milchprodukten gewonnen wurden, enthalten. Die vorteilhafte Wirkung dieser Nahrungszusammensetzung wird auf die Blockierung von entzündungsfördernden Zytokinen wie TNF-alpha, IL-6 zurückgeführt. Individuelle Peptide, die für diese Wirkungen verantwortlich sind, werden nicht genannt. Die Hydrolyse erfolgt also rein empirisch-phänomenologisch, ohne Angabe einer chemisch-analytischen Zielgröße (z. B. kein Hydrolysegrad, keine Molmassenbereiche, keine individuellen Peptide).

Patent WO 2007/004876 A2 betrifft zwar ACE-inhibierende Peptide aus Molkenproteinen, konzentriert sich dabei jedoch auf höhermolekulare Peptide (Oligopeptide mit mehr als 2 bis 14 Aminosäuren). Die Dipeptide Ile-Trp und Trp-Leu sind jedoch nicht genannt.

Die Publikation von Mullaly, M. M. et al. (Biol. Chem. Hoppe-Seyler, Vol. 377, pp. 259-260, April 1996) betrifft synthetische Peptide mit ACE-hemmender Wirkung, die bestimmten Sequenzbereichen von alpha-Lactalbumin und beta-Lactoglobulin entsprechen. Diese Sequenzen umfassen jedoch nicht die Dipeptide Ile-Trp und Trp-Leu.

Der Abstract von Philanto-Leppälä A. et al. (Journal of Dairy Research (2000) 67 (1), pp. 53-64) betrifft generell die ACE-hemmende Wirkung von Molkenhydrolysaten.

Die bisher verwendeten Peptide sind primär ACE-inhibitorisch wirksam, eine Wirkung auf andere Zielsysteme (Endothelin converting Enzyme, Matrix-Metalloproteasen) und damit gefäßprotektive Breitbandwirkung ist nicht beschrieben. Weiterhin sind zahlreiche der bisher beschriebenen Peptide Oligopeptide, die aus 3 und mehr Aminosäuren bestehen. Dies bedingt eine ausgeprägte Hydrolyselabilität im Dünndarm und entsprechend schlechte orale Verfügbarkeit.

α-Lactalbumin fällt heute als Teil der Molke im Rahmen der Käseherstellung täglich im Tonnenmaßstab an. Eine umfassende Nutzung ist zurzeit nicht gegeben. Die Nutzbarmachung ("Veredelung") eines bislang in der Lebensmittelindustrie wenig genutzten Nahrungsproteins (α-Lactalbumin) zur Herstellung funktioneller Lebensmittel wäre daher höchst wünschenswert.

Neu und Gegenstand dieses Patentes ist der Einsatz von speziellen tryptophanhaltigen Peptiden (Hydrolysate) aus α-Lactalbumin, die eine ACE hemmende Wirkung aufweisen und als Inhaltstoffe für funktionelle Nahrungsmittel, Nahrungsergänzungsmittel bzw. pharmazeutische Präparationen verwendet werden können.

Der hier beschriebenen Erfindung liegt die Aufgabe zugrunde, hocheffiziente, d. h. stark ACE-hemmende und damit potenziell Blutdruck-senkende Peptide durch proteolytischen Abbau von α-Lactalbumin, einem bislang weitgehend ungenutzten Molkenprotein, zu generieren und als bioaktive Inhaltsstoffe für funktionelle Lebensmittel einzusetzen.

Der Erfindung liegt weiterhin die Aufgabe zugrunde, Produkte zur Verfügung zu stellen, die tryptophanhaltige Peptide enthalten, die neben einer Blutdrucksenkung aufgrund ACE-Hemmung zu einer darüber hinaus gehenden Gefäßprotektion durch Hemmung weiterer relevanter Zielsysteme (ECE, MMP) beitragen. Durch die hier beschriebene Erfindung wird eine breite Produktpalette funktionell wirksamer Lebensmittel erschlossen.

Überraschenderweise werden diese Aufgaben gelöst durch die erfindungsgemäßen Molkenproteinhydrolysate. Der Anmeldungsgegenstand wird in den Ansprüchen definiert.

Die Erfindung umfasst ein Molkenproteinhydrolysat mit ACE-hemmender und antihypertensiver Wirkung, das eine physiologisch wirksame Menge mindestens eines der bioaktiven tryptophanhaltigen Peptide mit einer Sequenz ausgewählt aus den SEQ ID-No. 2 bis 32 enthält. Das erfindungsgemäße Proteinhydrolysat ist erhältlich durch extensive Hydrolyse von Molkenproteinisolaten bzw. reinem α-Lactalbumin. Unter extensiver Hydrolyse im erfindungsgemäßen Sinn ist zu verstehen, dass die mittleren Molmassen der Peptide im Hydrolysat weniger als 4 kDa, bevorzugt weniger als 1000 Da, besonders bevorzugt weniger als 500 Da betragen. Dies entspricht einem Abbau der Proteine zu Di- bis Pentapeptiden. Die Hydrolyse wird dabei bevorzugt enzymatisch durchgeführt.

Als Ausgangsstoff für die Hydrolyse eignen sich sowohl Molke als auch handelsübliches Molkenpulver, Molkenproteinisolat, Molkenproteinpräparate oder aus Molke gewonnenes α-Lactalbumin, die industriell weiter prozessiert wurden durch Verfahrensschritte wie Mikrofiltration, isoelektrische Ausfällung, Chromatographie etc. Bevorzugt wird als Ausgangsstoff für die Hydrolyse α-Lactalbumin genutzt. Das Protein α-Lactalbumin ist in Kuhmilchmolke mit einem Anteil von etwa 20 % am Gesamtmolkenprotein enthalten und stellt somit nach dem ß-Lactoglobulin das zweithäufigste Molkenprotein in Kuhmilchmolke dar. Vorteilhaft macht das erfindungsgemäße Molkenproteinhydrolysat dieses bislang in der Lebensmittelindustrie wenig genutzte Nahrungsprotein nutzbar ("Veredelung") für die Herstellung funktioneller Lebensmittel. Durch die Hydrolyse dieser "Abfallverbindung" werden biologisch hochwirksame Peptide gewonnen, die als ACE-Hemmer für die prophylaktische und pharmakologische Beeinflussung des Bluthochdrucks nutzbar sind.

Überraschenderweise werden in dem erfindungsgemäßen Molkenproteinhydrolysat durch Hydrolyse bioaktive tryptophanhaltige Peptide mit einer Länge von zwei bis fünf, bevorzugt zwei bis drei, besonders bevorzugt zwei Aminosäuren aus der Primärsequenz von α-Lactalbumin freigesetzt. Die erfindungsgemäßen bioaktiven tryptophanhaltigen Peptide enthalten die Aminosäure Tryptophan an endständiger Position, d. h. entweder als C- oder als N-terminale Aminosäure. Bevorzugt ist das Tryptophan C-terminal positioniert.

Die Sequenzen der erfindungsgemäßen bioaktiven Peptide ergeben sich also aus der Primärsequenz des α-Lactalbumin und umfassen den betreffenden Tryptophanrest (W26, W60, W104 und W118) und 1 bis 4 Aminosäuren, die sich in der α-Lactalbumin-Sequenz entweder N- oder C-terminal zum betreffenden Tryptophanrest befinden.

Die Primärsequenz von bovinem α-Lactalbumin (UniProtKB/Swiss-Prot **P00711** [LALBA_BOVIN]) entspricht der SEQ ID-No. 1:

Bei Verwendung von Kuhmilchmolke zur Herstellung der erfindungsgemäßen Hydrolysate ergeben sich die Sequenzen der erfindungsgemäßen bioaktiven Peptide aus der Primärsequenz des bovinen α-Lactalbumin und umfassen 1 bis 4 Aminosäuren entweder N- oder C-terminal von einem der enthaltenen Tryptophane (W26, W60, W104 und W118) und das betreffende Tryptophan.

Die erfindungsgemäßen bioaktiven tryptophanhaltigen Peptide umfassen also bevorzugt die Dipeptide mit den SEQ ID-No. 2 - 8, die Tripeptide mit den SEQ ID-No. 9 - 16, die Tetrapeptide mit den SEQ ID-No. 17 - 24 und die Pentapeptide mit den SEQ ID-No. 25 - 32.

Die Sequenzen der erfindungsgemäßen tryptophanhaltigen Peptide sind in der folgenden Tabelle aufgelistet:

| SEQ ID-No. | Sequenz (3-Buchstaben-Code) | Sequenz (1-Buchstabencode) |
|---|---|---|
| 2 | Glu-Trp | E**W** |
| 3 | Trp-Val | **W**V |
| 4 | Ile-Trp | I**W** |
| 5 | Trp-Cys | **W**C |
| 6 | Tyr-Trp | Y**W** |
| 7 | Trp-Leu | **W**L |
| 8 | Gln-Trp | Q**W** |
| 9 | Pro-Glu-Trp | PE**W** |
| 10 | Trp-Val-Cys | **W**VC |
| 11 | Lys-Ile-Trp | KI**W** |
| 12 | Trp-Cys-Lys | **W**CK |
| 13 | Asn-Tyr-Trp | NY**W** |
| 14 | Trp-Leu-Ala | **W**LA |
| 15 | Asp-Gln-Trp | DQ**W** |
| 16 | Trp-Leu-Cys | **W**LC |
| 17 | Leu-Pro-Glu-Trp | LPE**W** |
| 18 | Trp-Val-Cys-Thr | **W**VCTT |
| 19 | Asn-Lys-Ile-Trp | NKI**W** |
| 20 | Trp-Cys-Lys-Asp | **W**CKD |
| 21 | Ile-Asn-Tyr-Trp | INY**W** |
| 22 | Trp-Leu-Ala-His | **W**LAH |
| 23 | Leu-Asp-Gln-Trp | LDQ**W** |
| 24 | Trp-Leu-Cys-Glu | **W**LCE |
| 25 | Ser-Leu-Pro-Glu-Trp | SLPE**W** |
| 26 | Trp-Val-Cys-Thr-Thr | **W**VCTT |
| 27 | Asn-Asn-Lys-Ile-Trp | NNKI**W** |
| 28 | Trp-Cys-Lys-Asp-Asp | **W**CKDD |
| 29 | Gly-Ile-Asn-Tyr-Trp | GINY**W** |
| 30 | Trp-Leu-Ala-His-Lys | **W**LAHK |
| 31 | Lys-Leu-Asp-Gln-Trp | KLDQ**W** |
| 32 | Trp-Leu-Cys-Glu-Lys | **W**LCEK |

Besonders bevorzugt sind in den erfindungsgemäßen Molkenproteinhydrolysaten das Dipeptid Ile-Trp, der bislang potenteste in Lebensmitteln nachgewiesene ACE-Inhibitor (IC₅₀ = 0,7 µM), und/oder das ebenfalls hochwirksame Dipeptid Trp-Leu (IC₅₀ = 10 µM) enthalten.

Die in dem erfindungsgemäßen Molkenproteinhydrolysat enthaltenen, bislang aus dem α-Lactalbumin nicht bekannten Dipeptide zeichnen sich durch starke ACE-Hemmung aus und besitzen die bislang höchste für Nahrungspeptide beschriebene Wirkung.

Die bioaktiven tryptophanhaltigen Peptide besitzen eine Strukturverwandtschaft mit ACE-Hemmern. Aufgrund dieser Strukturverwandtschaft kann eine umfassende physiologische Wirkung, d. h. Gefäßprotektion, Herzmassenreduktion, Regression der Gefäßwandhypertrophie, Schutz vor Herzinfarkt und Schlaganfall neben ACE-Hemmung bzw. damit verbundener Blutdrucksenkung abgeleitet werden. Diese Wirkung wurde auch durch Experimente an spontan-hypertensiven Ratten mit einem Molkenproteinhydrolysat, das diese Peptide enthält, nahegelegt.

Das erfindungsgemäße Molkenproteinhydrolysat ist charakterisiert durch den IC₅₀-Wert der ACE-Hemmung. Je niedriger der IC₅₀-Wert, desto höher ist die ACE-hemmende Wirkung. Das erfindungsgemäße Molkenproteinhydrolysat besitzt vorzugsweise einen IC₅₀ von 50 bis 100 mg Molkenproteinhydrolysat/Liter, bevorzugt von 20 bis 50 mg Molkenproteinhydrolysat/Liter, besonders bevorzugt von 2 bis 20 mg Molkenproteinhydrolysat/Liter.

Aufgrund der niedrigen IC₅₀-Werte und der starken ACE-Hemmung sind zudem sehr geringe Mengen an Peptidzusätzen (für Ile-Trp nur ca. 1/10, verglichen mit den oben genannten Tripeptiden Ile-Pro-Pro und Val-Pro-Pro) nötig, was vor dem Hintergrund möglicher sensorischer Aspekte vorteilhaft ist. Proteinhydrolysate erhalten nämlich durch die Hydrolyse meist einen bitteren Geschmack, welcher den Einsatz bei der Herstellung von Nahrungsprodukten, die zum Verzehr geeignet sind, oder Arzneimittel, die oral aufgenommen werden, deutlich einschränkt. Durch diesen bitteren Geschmack können Proteinhydrolysate nur in sehr begrenztem Umfang diesen Produkten beigefügt werden. Das erfindungsgemäße Molkenproteinhydrolysat ist jedoch bereits in kleinen Mengen hochwirksam und kann daher vorteilhaft für die Herstellung von Nahrungsprodukten und oral zu verabreichenden Arzneimitteln eingesetzt werden.

Die in dem erfindungsgemäßen Molkenprotein enthaltenen hochwirksamen Peptide bestehen bevorzugt aus hochgradig hydrophoben Aminosäuren wie zum Beispiel die Peptide mit den SEQ ID No. 3, 4, 7, 11, 14, 17 und 22.

Dies macht die tryptophanhaltigen Peptide vorteilhaft stabil gegen Hydrolyse, so dass selbst bei langen Hydrolysezeiten davon ausgegangen werden kann, dass das Hydrolysat wirksame Konzentrationen der erfindungsgemäßen tryptophanhaltigen Peptide enthält.

Die Erfindung umfasst weiterhin ein Verfahren zur Herstellung der erfindungsgemäßen Molkenproteinhydrolysate. Die Hydrolyse wird bevorzugt enzymatisch mit wenigstens den Enzymen Alcalase und Trypsin durchgeführt. Der Verdau mit den beiden Enzymen kann aufeinander folgen oder gleichzeitig durchführt werden. Das Enzym/Substrat-Verhältnis (g Enzym/g Substrat) liegt vorzugsweise zwischen 1:10 und 1:10.004.

Besonders bevorzugt werden noch Verdaue mit weiteren Enzymen angeschlossen, oder weitere Enzyme dem Verdau mit Alcalase und Trypsin zugesetzt. Bei den verwendeten Enzymen handelt es sich bevorzugt um Endoproteasen, besonders bevorzugt um die Enzyme Chymotrypsin, Pancreatin, Pepsin und CorolasePP.

Die Geschwindigkeit der Hydrolyse hängt von den verwendeten Enzymen sowie von deren Konzentration und der Hydrolysetemperatur ab. Auch weitere Parameter können die Geschwindigkeit der Hydrolyse beeinflussen, wie beispielsweise der pH-Wert oder die chemische Zusammensetzung des Hydrolysesubstrats.

Die Hydrolyse wird so lange durchgeführt, bis mehr als 50 % des enthaltenen Proteins eine Molmasse von kleiner 4 kDa, bevorzugt kleiner 1 kDa, besonders bevorzugt kleiner 500 Da, besitzt. Das entspricht einem Abbau der Proteine zu Di- bis Pentapeptiden. Die erfindungsgemäßen Dipeptide Ile-Trp und Trp-Leu besitzen beispielsweise eine Molmasse von ca. 317 Da. Durch die ausreichend lange Durchführung der Hydrolyse wird sichergestellt, dass ein möglichst hoher Anteil der bioaktiven tryptophanhaltigen Peptide in dem erfindungsgemäßen Molkenproteinhydrolysat erreicht wird. Die enzymatische Hydrolyse wird bei einer Temperatur zwischen 10 °C und 80 °C, bevorzugt zwischen 30 °C und 70 °C, besonders bevorzugt zwischen 50 °C und 60 °C durchgeführt. Der Fortschritt der Hydrolyse wird dabei beispielsweise durch Gelpermeationschromatographie (GPC) überwacht und nach Erreichen des gewünschten Hydrolysegrads durch Inaktivierung der Enzyme, bevorzugt durch Erhitzen auf eine Temperatur vom 80 °C bis 100 °C, abgestoppt. Der Hydrolyseschritt (Inkubationszeit) dauert vorzugsweise 12 h bis 36 h, bevorzugt 18 h bis 30 h, besonders bevorzugt 20 h bis 28 h.

Die Erfindung umfasst auch die Verwendung von Molkenprotein zur Herstellung des erfindungsgemäßen Molkenproteinhydrolysats. Die erfindungsgemäßen bioaktiven tryptophanhaltigen Peptide sind in der Primärsequenz des Molkenproteins α-Lactalbumin enthalten und werden durch die enzymatische Hydrolyse aus diesem freigesetzt. Bevorzugt wird daher für die Herstellung des Molkenproteinhydrolysats mit α-Lactalbumin angereichertes Molkenprotein, besonders bevorzugt auch α-Lactalbumin selbst als Molkenprotein, für die Herstellung des Molkenproteinhydrolysats verwendet.

Die Erfindung umfasst weiterhin ein Verfahren zur Herstellung bioaktiver tryptophanhaltiger Peptide mit einer Sequenz ausgewählt aus den SEQ ID-No. 2 bis 32 mit ACE-hemmender und antihypertensiver Wirkung. Das Verfahren umfasst dabei die Hydrolyse von Molkenprotein und die anschließende Gewinnung der bioaktiven tryptophanhaltigen Peptide aus dem Molkenproteinhydrolysat. Bevorzugt wird die Hydrolyse dabei gemäß dem in den Ansprüchen 7 bis 10 offenbarten Verfahren ausgeführt.

Als Ausgangsstoff für die Hydrolyse eignen sich Molke, handelsübliches Molkenpulver, Molkenproteinisolat, Molkenproteinpräparate oder aus Molke gewonnenes α-Lactalbumin, die industriell weiter prozessiert wurden durch Mikrofiltration, isoelektrische Ausfällung, Chromatographie etc. Die Erfindung umfasst daher auch die Verwendung von Molkenprotein zur Herstellung der bioaktiven tryptophanhaltigen Peptide.

Für die Herstellung der erfindungsgemäßen Hydrolysate eignet sich im Prinzip jede Art von Säugetiermilch, solange sie einen ausreichenden Gehalt an α-Lactalbumin (ca. 100 mg/liter) in der Molke enthält. Bevorzugt wird jedoch Kuhmilchmolke verwendet.

Die Gewinnung der bioaktiven tryptophanhaltigen Peptide erfolgt über Standardtechniken der präparativen Chemie, wie etwa Extraktion mit organischen Lösungsmitteln, Ultrafiltration oder bevorzugt durch präparative RP-HPLC (Reversed Phase-Hochleistungsflüssigkeitschromatographie).

Die aus dem Molkenproteinhydrolysat isolierten tryptophanhaltigen Peptide können Nahrungsprodukten zugesetzt werden. Zu den Nahrungsprodukten gehören Lebensmittel wie Nahrungsmittel und Genussmittel, und Nahrungsergänzungsmittel. Schließlich eignen sich die neuen wirksamen tryptophanhaltigen Peptide aus α-Lactalbumin zur pharmazeutischen Herstellung von ACE-Inhibitoren.

Die Erfindung umfasst auch die Verwendung des erfindungsgemäßen Molkenproteinhydrolysats zur Herstellung eines Nahrungsprodukts. Weiterhin umfasst die Erfindung die Verwendung des erfindungsgemäßen Molkenproteinhydrolysats zur Herstellung eines blutdrucksenkenden Arzneimittels.

Die Erfindung umfasst ebenfalls die Verwendung eines gemäß Anspruch 7 hergestellten bioaktiven tryptophanhaltigen Peptids zur Herstellung eines Nahrungsprodukts.

Schließlich umfasst die Erfindung die Verwendung eines gemäß Anspruch hergestellten bioaktiven tryptophanhaltigen Peptids gemäß Anspruch 7 zur Herstellung eines blutdrucksenkenden Arzneimittels. Das erfindungsgemäße Arzneimittel eignet sich speziell zur oralen Verabreichung und kann in jeder für die orale Verabreichung geeigneten Formulierung zubereitet werden. Geeignete Verabreichungsformen sind beispielsweise Pulver, Instantpulver, Presslinge, Granulate, Tabletten, Brausetabletten, Kapseln, Dragees, Sirup oder dergleichen.

Die hier beschriebenen, die bioaktiven tryptophanhaltigen Peptide enthaltenden, Molkenproteinhydrolysate können direkt Nahrungsprodukten zugesetzt werden (z. B. Milch- und Molkengetränken, Fruchtsäften, Erfrischungsgetränken).

Die Molkenproteinhydrolysate können auch durch Gefrier- oder Sprühtrocknung getrocknet und zu Pulver verarbeitet werden. Sie sind dabei als Inhaltsstoffe, die konfektioniert und/oder in Rezepturen mit anderen Nahrungsmittelergänzungsstoffen, wie Vitaminen, Mineralstoffe und/oder Spurenelemente verwendet werden, einsetzbar. Die Pulver eignen sich als Zusatz zu Nahrungsprodukten und darüber hinaus zur Verwendung als lose Pulver, um Granulate, Tabletten, Kapseln, Pastillen, Süßigkeiten und Flüssigkeiten zu erzeugen. Gegebenenfalls können dem Pulver Hilfsstoffe und Bindemittel zugegeben werden.

Das erfindungsgemäße Molkenproteinhydrolysat wird vorzugsweise als Instantprodukt (lösliches Getränkepulver, wie beispielsweise ein Kakao-, Kaffee- oder Teeprodukt-Getränkepulver, Sirup, Konzentrat, Brausepulver oder -tablette) angeboten, das in Portionspackungen von etwa 5 bis 50 g, bevorzugt 5 bis 10 g, abgefüllt ist und beispielsweise in 250 ml Wasser oder Fruchtsaft aufgelöst werden kann. Das Instantpulver enthält das erfindungsgemäße Molkeproteinhydrolysat vorzugsweise in einer Menge von 50 Gew.-% bis 98 Gew.-%, bevorzugt von 70 Gew.-% bis 95 Gew.-%, besonders bevorzugt von 80 Gew.-% bis 92 Gew.-% bezogen auf das Gesamtgewicht des Instantpulvers.

Das Molkenproteinhydrolysat kann dabei selbst als Trägermaterial für die weiteren Komponenten des Instantpulvers dienen; das Instantpulver kann jedoch auch weitere übliche Trägerstoffe enthalten. Zusätzlich zu dem Molkenproteinhydrolysat enthält das Instantpulver gegebenenfalls Mineralstoffe, Spurenelemente, Vitamine, natürliche und künstliche Süßungsmittel, Aromen, Säuerungsmittel, Carbonatverbindungen, Farbstoffe, Konservierungsmittel, Antioxidantien, Stabilisatoren und/oder sonstige Lebensmittelzusatzstoffe.

Die Herstellung von Instantgetränkepulvern ist dem Fachmann geläufig und erfolgt nach Standardprozeduren.

Analoge Verfahren können für die Substitution der Peptide in Trockenfutter/Pellets für Tiere verwendet werden.

Die Erfindung umfasst weiterhin ein Nahrungsprodukt, enthaltend das erfindungsgemäße Molkenproteinhydrolysat oder mindestens ein nach dem erfindungsgemäßen Verfahren hergestelltes bioaktives tryptophanhaltiges Peptid.

Bei den Nahrungsprodukten handelt es sich beispielweise um Backwaren, insbesondere Brot, Kleingebäck, Feingebäck, Dauer-Backwaren, Keksprodukte und Waffeln, um Desserts, insbesondere Pudding, Creme und Mousse, um Brotaufstriche, Margarine-Erzeugnisse, Backfette, um Obstprodukte, insbesondere Konfitüren, Marmeladen, Gelees, Obstkonserven, Fruchtmark, Fruchtsäfte, Fruchtsaft-Konzentrate, Fruchtnektar und Fruchtpulver, um Gemüseerzeugnisse, insbesondere Gemüsekonserven, Gemüsesäfte und Gemüsemark, um Cerealien, Müsli und Cerealien-Mischungen, oder um Süßwaren wie Schokoladen, Bonbons, Kaugummi, Dragees, Lakritzen, Schaumzuckerwaren, Flocken und Nougaterzeugnissen.

Bei diesem Nahrungsprodukt handelt es sich bevorzugt um ein Milcherzeugnis oder Milchprodukt. Das Milchprodukt ist besonders bevorzugt ausgewählt aus der Gruppe enthaltend Milch, aus Milch hergestellte Brotaufstriche, Milch- und Molkengetränke, Joghurt und Joghurtgetränke, und sonstige aus Milch hergestellte Erfrischungsgetränke sowie Speiseeis, Frischkäse-, Käse-, Butter-, Kefir-, Quark, Sauermilch-, Buttermilch-, Sahne-, Kondensmilch-, Trockenmilch-, Molke-, Milchzucker-, Milcheiweiß-, Milchhalbfett-, Molkenmisch- oder Milchfett-Produkte oder Zubereitungen.

Das Nahrungsprodukt enthält gegebenenfalls Ergänzungs-, Hilfs- und/oder Süßstoffe. Ergänzungs- oder Hilfsstoffe sind bevorzugt ausgewählt aus der Gruppe bestehend aus Aromastoffen, z. B. Vanillin, Farbstoffen, Geschmacksstoffen, Emulgatoren, z. B. Lecithin, Verdickungsmitteln, z. B. Pektin, Johannisbrotkernmehl oder Guargummi, Antioxidationsmitteln, Konservierungsmitteln, Triglyzeriden und natürlichen oder synthetischen Vitaminen, z. B. Vitamin A, Vitamin B₁, Vitamin B₂, Vitamin B₆, Vitamin B₁₂, Vitamin B-Komplex, Vitamin C, Vitamin D, Vitamin E und/oder Vitamin K. Süßstoffe sind bevorzugt ausgewählt aus Sucralose, Natriumcyclamat, Acesulfam K, Aspartam, Saccharin, Acesulfam, Cyclamat, Thaumatin und Neohesperidin.

Die nutritive Zufuhr antihypertensiv wirksamer Inhaltsstoffe eignet sich vorteilhaft zum komplementären Einsatz zur pharmakologischen Behandlung und ergänzt die Palette unterstützender ("life style") Verfahren wie z. B. Einschränkung der Kochsalzzufuhr, Sport, Yoga und weitere diätetische Maßnahmen. Die aus dem proteolytischen Abbau von Molkenprodukten stammenden ACE-inhibierenden Peptide tragen daher in funktionellen Lebensmitteln wesentlich zur Vorbeugung und zur unterstützenden Behandlung des Bluthochdrucks bei.

Schließlich eignen sich die neuen wirksamen tryptophanhaltigen Peptide aus α-Lactalbumin zur pharmazeutischen Herstellung von ACE-Inhibitoren und sind so in der Pharmakotherapie des Bluthochdrucks einschließlich Gefäßwand-Remodeling, zur Herzmassenreduktion, Verbesserung der Durchblutungsreserve und zur Absenkung des Herzinfarktrisikos und des Schlaganfallrisikos als Mono- und als Kombinationspräparat einsetzbar.

Die Erfindung umfasst daher auch ein blutdrucksenkendes Arzneimittel, enthaltend das erfindungsgemäße Molkenproteinhydrolysat oder mindestens ein nach dem erfindungsgemäßen Verfahren hergestelltes bioaktives tryptophanhaltiges Peptid. Das blutdrucksenkende Arzneimittel kann dabei noch weitere Wirkstoffe, bevorzugt weitere ACE-Inhibitoren oder andere blutdrucksenkende Wirkstoffe enthalten. Das Arzneimittel kann außerdem Lösungsmittel, Lösungsvermittler, Antioxidantien, Resorptionsverstärker und andere Zusatzstoffe enthalten. Das Arzneimittel kann in flüssiger oder fester Form vorliegen und wird vorzugsweise oral verabreicht.

Die erfindungsgemäßen Molkenproteinhydrolysate oder die nach dem erfindungsgemäßen Verfahren hergestellten bioaktiven tryptophanhaltigen Peptide eignen sich desweiteren als Zusatz zu Tiernahrungsmitteln, als Tiernahrungsergänzungsmittel (Snack) oder als Tierarzneimittel. Die Molkenproteinhydrolysate oder die nach dem erfindungsgemäßen Verfahren hergestellten bioaktiven tryptophanhaltigen Peptide können dabei direkt oder in getrockneter Form festen Futtermitteln, Kraftfutter, Konzentratkraftfutter, Futterergänzungsmitteln, Trinkwasser, Lecksteinen oder Vorgemischen für derartige Zusammensetzungen zugesetzt werden. Die Molkenproteinhydrolysate können auch als Pulver vom Tierhalter unter das Tierfutter oder das Trinkwasser gemischt werden. Gegebenenfalls enthält das Pulver neben dem Molkenproteinhydrolysat Zusatz- und Hilfsstoffe. Insbesondere können weitere Zusatzstoffe ausgewählt sein aus der Gruppe der Mineralstoffe, Vitamine, Spurenelemente, Zucker, Malz, Melasse, Getreide, Kleie, Samen (insbesondere von Ölpflanzen), Proteine, Aminosäuren, Salze, Öle, Fette, Fettsäuren, Früchte, Fruchtbestandteile oder Fruchtextrakte oder Mischungen daraus.

Auf diese Weise kann das Futter für Haus- und Nutztiere, wie beispielsweise Pferde, Kühe, Esel, Schafe, Ziegen, Hunde, Katzen, Schweine, Hasen, Kaninchen, Meerschweinchen, Hamster oder Vögel ebenso mit den erfindungsgemäßen Molkenproteinhydrolysaten angereichert werden wie das von Zootieren bzw. Exoten wie z. B. Affen, Zebras, Antilopen, Giraffen, Raubkatzen, Büffel, Nagern, und vielen mehr, ohne auf die genannten Tiere beschränkt zu sein.

Es können bis zu 50 Gew.-% des erfindungsgemäßen Molkenproteinhydrolysats in eine Futterrezeptur (als Futtermittel oder als Ergänzungsmittel) eingearbeitet werden. Üblicherweise wird das Molkenproteinhydrolysat jedoch in einem Bereich von 0,01 bis 20 Gew.-%, bevorzugt 0,05 bis 10 Gew.-%, besonders bevorzugt 0,05 bis 5 Gew.-% in die Zusammensetzungen eingearbeitet. Dabei ist die Menge des einzuarbeitenden Molkenproteinhydrolysats und dessen Teilchengröße abhängig von der jeweils zu berücksichtigenden Tierart.

Wird das Molkenproteinhydrolysat - ggf. zusammen mit wenigstens einem der weiteren oben genannten Zusätze - als Futterergänzungsmittel bereitgestellt, erfolgt dies entweder unmittelbar als Mischung der Bestandteile ohne eine vorhergehende Konfektionierung oder in Form eines Granulats, von Presslingen, Pellets, Pulver, Dragees, Sirup, einer Suspension oder irgendeiner anderen geeigneten Verabreichungsart.

Vorzugsweise wird das Futterergänzungsmittel, das das erfindungsgemäße Molkenproteinhydrolysat enthält, in einer Form bereitgestellt, die es dem Tierhalter ermöglicht, eine individuelle Dosierung an das jeweilige Tier zu verabreichen. Dies ist beispielsweise bei einem Pulver oder einem Granulat, aber auch bei Dragees oder Pellets in besonderer Weise gegeben.

Dabei zeigt
**Fig. 1** GPC-Chromatogramme der Molmassenverteilung des nativen (Milei60 0 h) sowie des hydrolysierten Molkenproteins nach 4 h (Milei60 4 h) und 48 h (Milei60 48 h) Inkubationszeit,
**Fig. 2** zeigt die Zunahme der niedermolekularen (lmw) sowie Abnahme der hochmolekularen Massen (hmw) mit zunehmender Hydrolysezeit,
**Fig. 3** zeigt den IC₅₀-Wert der ACE-Hemmung des Molkenproteinpulvers Milei60 in Abhängigkeit der Hydrolysezeit,
**Fig. 4** zeigt den systolischen Blutdruck spontan hypotensiver Ratten zu Beginn (0 Wochen), nach 7 und 15 Wochen Fütterungsstudie. Verabreicht wurde ein Präparat, das erfindungsgemäße Peptide enthält (PP), Futter mit intaktem Molkenprotein (MP) sowie zur Kontrolle ein Rattenfutter mit Captopril, einem bekannten ACE-Inhibitor, sowie ein Placebo. PP führte zu einer mit Captopril vergleichbaren Blutdrucksenkung.

### Ausführungsbeispiel 1: Enzymatische Hydrolyse des Molkenproteinpulvers Milei60

Das Molkenprotein Milei60 wurde mittels proteolytisch wirkender Enzyme, dem aus dem Schweinepankreas gewonnenen Trypsin (EC 3.4.21.4), sowie zweier weiterer Proteasen, der Alcalase® (*Bacillus licheniformis*) und Flavourzyme (*Aspergillus oryzae*) enzymatisch hydrolysiert. Die nachfolgende Tabelle stellt die Durchführung der Hydrolyse dar.

**Tab. 1: Durchführung der enzymatischen Hydrolyse des Molkenproteinpulvers Milei60**

| **Verfahrensschritte** | **Molkenproteinlösung** |
|---|---|
| | Milei60 10g/50 ml bidest. Wasser |
| Einstellung des pH-Werts mit 3 N NaOH | pH = 7,0 |
| Zugabe Enzym | Trypsin: 0,0015 g (2,338 U) |
| | Alcalase: 200 µl (0,07 U) |
| | Flavourzyme: 100 µl (7,5 U) |
| Inkubationsdauer | 10 min, 30 min, 1 h, 2 h, 4 h, 8 h, 12 h, 30 h, 48 h |
| Hydrolysetemperatur | 55 °C im Wasserbad |
| Abkühlung | < 10 °C innerhalb von 10 min |
| Inaktivierung | Erhitzung bei 100 °C für 10 min im Trockenschrank |

Der Grad der Hydrolyse wurde über die Molmassenverteilung der unterschiedlich lang inkubierten Proben durch Gel-Permeationschromatographie (GPC) analysiert. Die Ermittlung der Molmassen erfolgte anschließend durch den Vergleich mit Standards. Kalibriert wurde im Bereich von 89,1 Da (Alanin) bis 25,0 kDa (Chymotrypsinogen) (siehe auch Tab. 2). Zur Bestimmung der Ausschlussgrenze diente Dextran-Blau. Aufgrund der Unterschiede im Absorptionsvermögen wurden verschiedene Konzentrationen der Standardsubstanzen verwendet. Nachfolgend sind die für die Kalibrierung nötigen Standardsubstanzen sowie ihre Molmassen und für die Chromatographie verwendete Konzentrationen und Retentionszeiten tabellarisch zusammengefasst.

**Tab. 2: Zur GPC-Kalibrierung verwendete Standardsubstanzen mit entsprechenden Molmassen [Da], Retentionszeiten [min] und eingesetzten Konzentrationen [mg/ml]**

| **Standard** | **Molmasse [Da]** | **Konzentration [mg/ml]** | **Retentionszeit [min]** |
|---|---|---|---|
| Dextran-Blau | 1000000 | 1,0 | 14,21 |
| Chymotrypsinogen | 25000 | 0,6 | 16,20 |
| Ribonuclease A | 13700 | 0,8 | 17,49 |
| Bovine Insulin | 5733 | 5,0 | 20,08 |
| PTHIKWGD | 953,07 | 2,0 | 30,55 |
| LG | 188,23 | 2,0 | 32,24 |
| GG | 132,12 | 2,0 | 33,29 |
| L | 131,18 | 4,0 | 33,11 |
| A | 89,09 | 4,0 | 33,33 |

Zur Bestimmung des Hydrolysegrades wurden für alle Proben eine einheitliche Proteinkonzentration von 10 mg/ml Ansatz gewählt. Eluiert wurde mit einer Phosphatgepufferten Kochsalzlösung. Die Detektion der eluierenden Substanzen erfolgte bei 220 und 280 nm. Die zur Durchführung verwendeten Chemikalien sowie Parameter sind im Folgenden aufgezeigt.

**Tab. 3: GPC-Parameter**

| | |
|---|---|
| **Säule:** | Superdex Peptide HR 10/30 |
| **Einspritzvolumen:** | 20 µl |
| **Säulentemperatur:** | Raumtemperatur |
| **Detektion:** | UV-Detektor: 220 und 280 nm |
| **Fließmittel:** | Phosphat-gepufferte Kochsalzlösung: 10 mM Phosphat + 300 mM Natriumchlorid (pH 7,4) |
| | 1,47 g Na₂HPO₄ · 2 H₂O (8,28 mM), 0,23 g KH₂PO₄ (1,72 mM), 17,53 g NaCl (300 mM) und 0,20 g KCl (2,7 mM) in 800 ml Reinstwasser lösen, mit Reinstwasser auf 1,01 auffüllen, membranfiltrieren (der pH-Wert der Lösung beträgt automatisch 7,4 und muss daher nicht eingestellt werden) |
| **Flussrate:** | 0,5 ml/min |
| **Elutionssystem:** | isokratisch; 70 min |

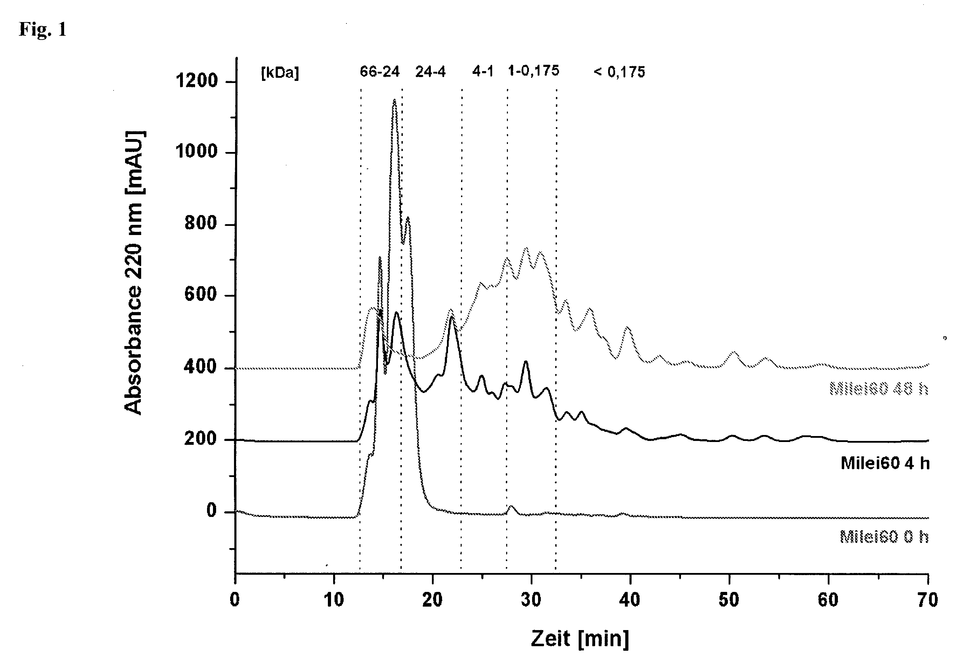

Der Hydrolyseverlauf des Molkenpulvers Milei60 wurde über 48 Stunden untersucht. Das Ausmaß des enzymatischen Verdaus wurde mittels GPC und anschließender UV-Detektion bei 220 nm und 280 nm bestimmt. Für eine Charakterisierung der Verteilung der Molmassen, wurden die Probenchromatogramme in fünf Abschnitte (66 - 24 kDa, 24 - 4 kDa, 4-1 kDa, 1 - 0,175 kDa und <0,175 kDa) eingeteilt, deren Bereiche in **Figur 1** gekennzeichnet sind. Dadurch ist es beispielsweise möglich anzugeben, inwieweit noch intaktes Protein vorliegt, oder ob während der Hydrolyse primär Aminosäuren, kurzkettige oder langkettige Peptide freigesetzt werden, was wiederum Informationen zur Bildung möglicher ACE-Inhibitoren liefern kann.

In **Figur 1** sind die Verteilungen der Molmassen einiger ausgewählter Hydrolysestufen (4h, 48h), sowie des unverdauten Molkenproteinpulvers (0h) dargestellt. Im Ausgangsprotein liegen, wie erwartet, mehrere hochmolekulare Verbindungen (66 - 24 kDa) vor. Dabei handelt es sich vorwiegend um die Hauptproteine α-Lactalbumin, das zum Großteil in seiner monomeren Form vorliegt, und β-Lactoglobulin. Letzteres trägt den größten Anteil an den Proteinen in Milei60 und liegt überwiegend als Dimer vor.

Aber auch weitere Molkenproteine wie beispielsweise das Bovine Serum Albumin (BSA), welches im vordersten Bereich eluiert, oder auch glycosylierte Formen der Molkenproteinkomponenten, werden mit dieser Methode detektiert. Kleinere Substanzen liegen nicht bzw. in nicht relevantem Maße in dem Produkt vor. Bereits nach nur zehn Minuten (siehe Tab. 4) hat ein relativ starker Abbau der Molkenproteine, im besonderen des α-Lactalbumin, stattgefunden, der sich im Laufe des enzymatischen Verdaus kontinuierlich fortsetzt, jedoch selbst nach 48 Stunden nicht zu einer vollständigen Hydrolyse führt. Im Laufe der Hydrolysezeit verschiebt sich der Anteil der Verbindungen immer mehr in den niedermolekularen Bereich, wo vorwiegend Dipeptide sowie freie Aminosäuren vorliegen. Zur besseren Veranschaulichung wurden die Verteilungen der Molmassen in den Hydrolysaten, die sich aus den prozentualen Flächenanteilen des GPC-Chromatogramms ergeben, in Tab. 4 bilanziert.

**Tab. 4: Prozentuale Flächenanteile entsprechender Molmassenbereiche der unterschiedlich lang hydrolysierten Molkenprotein-Proben**

| | **Prozentualer Flächenanteil der entsprechenden Molmassenbereiche** | | | | |
|---|---|---|---|---|---|
| **Probe** | **66 - 24 kDa** | **24 - 4 kDa** | **4 - 1 kDa** | **1-0,175 kDa** | **< 0,175 kDa** |
| nativ | 47,9 | 39,6 | 1,0 | 1,4 | 1,6 |
| 10 min | 30,8 | 37,5 | 11,7 | 12,7 | 7,2 |
| 30 min | 29,3 | 36,9 | 12,1 | 13,5 | 8,2 |
| 1 h | 28,0 | 34,7 | 12,8 | 15,5 | 9,0 |
| 2 h | 27,4 | 34,4 | 13,6 | 15,5 | 9,1 |
| 3 h | 26,9 | 33,6 | 14,1 | 15,9 | 9,6 |
| 4 h | 19,4 | 29,6 | 17,4 | 20,1 | 13,5 |
| 8 h | 18,1 | 26,1 | 19,3 | 22,3 | 14,2 |
| 12 h | 18,0 | 23,0 | 19,3 | 23,3 | 16,4 |
| 24 h | 13,8 | 15,0 | 21,5 | 29,9 | 19,8 |
| 30 h | 11,1 | 13,4 | 22,1 | 31,9 | 21,5 |
| 48 h | 6,4 | 8,2 | 28,9 | 33,4 | 23,1 |

Auch hier wird die stetige Verschiebung von dem hochmolekularen in den niedermolekularen Bereich während andauernder Hydrolyse sichtbar, wobei ein vollständiger Abbau nicht erreicht wird. Den größten Anteil nimmt schließlich nach 48 Stunden enzymatischen Verdaus nicht der Bereich < 0,175 kDa, in dem vorwiegend freie Aminosäuren vorliegen, ein, sondern Verbindungen mit einer molaren Masse zwischen 1 und 0,175 kDa machen den höchsten Anteil im Hydrolysat aus.

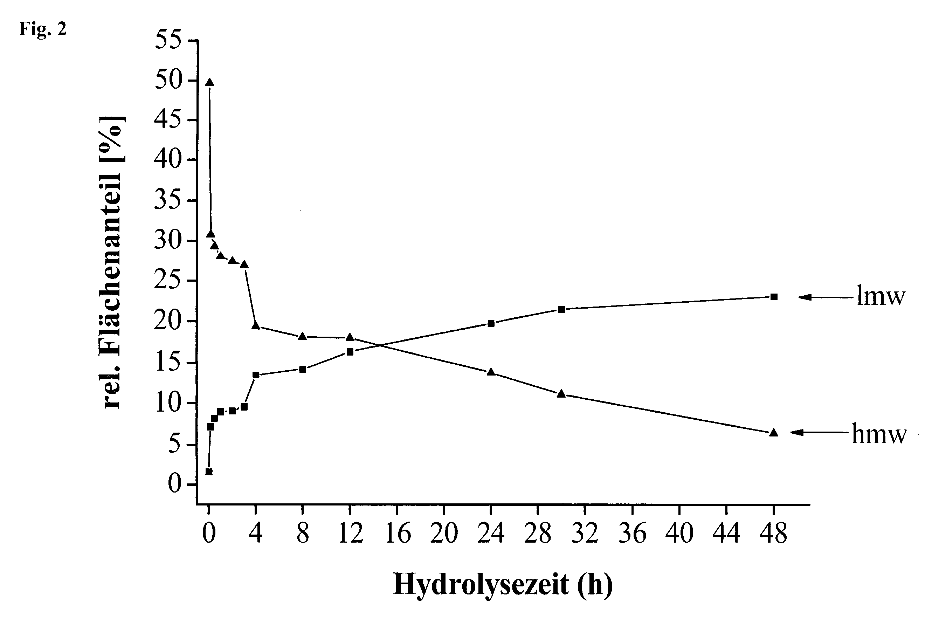

Der Abbau höhermolekularer (24 - 66 kDa) Verbindungen bzw. die Freisetzung niedermolekularer (< 0,175 kDa) Substanzen flacht nach einer Hydrolysezeit von 24 Stunden im Vergleich mit den vorhergehenden Stunden deutlich ab (siehe **Figur 2**).

Ab einer bestimmten Zeit fand kein linearer Abbau und damit Freisetzung kleiner Verbindungen statt, sondern nach ca. 18 Stunden stellte sich ein Plateau ein bzw. die Hydrolyse lief nur noch relativ langsam ab. Die stärkste Hydrolyse lief in den ersten Stunden ab und nach vier Stunden lagen mehr als 51 % der Peptide unter 4 kDa vor.

### Ausführungsbeispiel 2: Bestimmung des ACE-inhibierenden Effektes der Proteinhydrolysate

Die Bestimmung der ACE-Aktivität der Proteinhydrolysate erfolgte angelehnt an einem vom Cushman und Cheung 1979 beschriebenen Enzym-Test (Cushman, D. W. and Cheung, H.-S. Spectrophotometric Assay and Properties of Angiotensin-Converting Enzyme of Rabbit Lung, Biochemical Pharmacology, 1971, 20, 1637-1648). Allerdings wurden mehrere Modifizierungen durchgeführt. Bei dem verwendeten ACE handelt es sich um das Enzym aus der Kaninchenlunge, welches über Sigma-Aldrich bezogen wurde. Als Substrat diente das N-benzoyl-glycyl-L-histidyl-L-leucin (HHL), welches als Analoges des natürlichen Substrates Angiotensin I gilt. ACE katalysiert die Hydrolyse zu L-histidyl-L-leucin (HL) und N-benzoylglycin (Hippursäure):

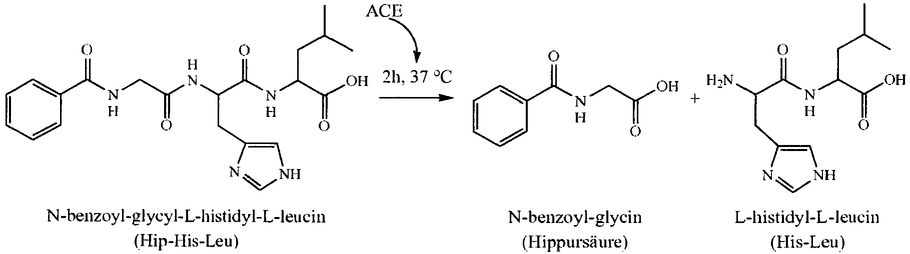

Da Hippursäure UV-aktiv (λₘₐₓ=228 nm) ist, kann über deren Gehalt, welcher mittels RP-HPLC bestimmt wird, der Umsatz und damit die Enzymaktivität definiert werden. Mit Hilfe einer Konzentrationsreihe der jeweiligen Inhibitorlösung kann die Inhibitorkonzentration ermittelt werden, die zur Senkung der ACE-Aktivität um die Hälfte nötig ist (IC₅₀).

In Tab. 5 sind die nötigen Reagenzien für die Durchführung des Aktivitäts-Tests beschrieben:

**Tab. 5: Reagenzien für die Bestimmung der ACE-Aktivität**

| | |
|---|---|
| Puffer: | 50 mM HEPES + 300 mM Natriumchlorid (pH 8,3 bei 37 °C) |
| | 2,383 g HEPES und 3,506 g NaCl in 190 ml Reinstwasser lösen, auf 37 °C erwärmen, pH-Wert der Lösung mit NaOH auf 8,3 einstellen, mit Reinstwasser auf 200 ml auffüllen |
| Substrat: | 5 mM Hip-His-Leu in HEPES-Puffer |
| Enzym: | ACE-Lösung |
| | 0,25 U in 4 ml bidest. Wasser lösen |
| Salzsäure: | 1N 8,3 ml 37 %ige HCl in Reinstwasser lösen, mit Reinstwasser auf 100 ml auffüllen |

Alle Untersuchungslösungen wurden in bidest. Wasser gelöst.

Tab. 6 zeigt die Durchführung des ACE-Aktivitätstests. In jeder Messreihe wurden zwei Enzym-Blindwerte mitgeführt, in denen an Stelle von Probenlösung das gleiche Volumen bidest. Wasser zugegeben wurde. Der Blindwert entspricht einer 100 %igen ACE-Aktivität. Die Berechnung der IC₅₀-Werte erfolgte über das Softwareprogramm SigmaPlot 5.0.

**Tab. 6: Durchführung des ACE-Aktivitätstests**

| | Blindwert | Probe |
|---|---|---|
| Substratzugabe (HHL) | 100 µl | |
| Zugabe der Probelösung | 25 µl bidest. Wasser | 25 µl Probelösung |
| Temperieren des Ansatzes | 10 min bei 37 °C | |
| Enzymzugabe | 20 µl ACE-Lösung (1,25 mU) | |
| Inkubation | 2 h bei 37 °C | |
| Abstoppen der Reaktion | 125 µl 1 N HCl | |

In Tab. 7 sind die RP-HPLC-Parameter zur Quantifizierung der Hippursäure, sowie die für die Kalibrierung benötigte Stammlösung mit entsprechenden Verdünnungen, aufgelistet:

**Tab. 7: RP-HPLC-Parameter zur Quantifizierung der Hippursäure**

| | |
|---|---|
| Stammlösung: | 1 mM Hippursäurelösung: 17,92 mg Hippursäure in Reinstwasser lösen und anschließend 100 ml auffüllen. |
| | Kalibrierlösungen: 0,005; 0,01; 0,025; 0,05; 0,1 mM |
| Säule: | Supersphere 100, RP 18 endcapped; 125 x 4,6 mm, Packungsdurchmesser 4 µm |
| Injektionsvolumen: | 20 µl |
| Säulentemperatur: | 25 °C |
| Flussrate: | 1,0 ml / min |
| Detektion: | UV: 228 nm |
| Elutionssystem: | Eluent A: 10 mM Kaliumdihydrogenphosphatlösung (pH 3,0) 1,3609 g KH₂PO₄ in 800 ml Reinstwasser lösen, pH-Wert der Lösung mit H₃PO₄ auf 3,0 einstellen, mit Reinstwasser auf 1 l auffüllen, membranfiltrieren |
| | Eluent B: 100 % Methanol (HPLC grade), entgast |
| Gradient: | Siehe Tab. 8 |

Tab. 8 zeigt den Gradienten, der zur Bestimmung des Hippursäuregehalts mittels RP-HPLC verwendet wurde.

**Tab. 8: RP-HPLC Gradientenparameter zur Bestimmung des Hippursäuregehalts**

| Zeit [min] | Eluent A [%] |
|---|---|
| 0 | 85 |
| 5 | 60 |
| 7 | 20 |
| 9 | 20 |
| 10 | 85 |
| 11 | 85 |

Neben den unterschiedlich lang inkubierten Proben wurde auch eine Blindwertprobe analysiert, die alle verwendeten Enzyme und das Molkenproteinpulver in gleicher Konzentration wie die Hydrolyseproben enthielt. Allerdings wurde nach Zugabe aller Substanzen sofort die enzymatische Wirkung durch eine 10minutige Inkubation bei 100 °C inaktiviert. Ebenso wurden eventuelle Effekte auf das ACE durch die Einzelverbindungen getestet. Dafür wurden die Enzyme inaktiviert und einzeln auf eine mögliche ACE-Hemmung untersucht. Genauso wurde mit dem Molkenproteinpulver verfahren. Auch nicht thermisch behandeltes Molkenproteinpulver wurde auf eine inhibierende Wirkung hin untersucht. Für die Bestimmung des IC₅₀-Wertes der Hydrolyseproben wurden mehrere Konzentrationen in dem ACE-Aktivitätstest eingesetzt. Ausgegangen wurde von einer Proteinkonzentration von 1000 mg Protein/l. Diese Stammlösung wurde 1:2, 1:5, 1:10, 1:20, 1:50, 1:100 verdünnt und alle Lösung hinsichtlich ihres ACE-hemmendes Effektes untersucht. Zur Auswertung wurde auch hier mit SigmaPlot 5.0 gearbeitet.

Um einen möglichen Einfluss der eingesetzten Hydrolyseenzyme Trypsin und Alcalase sowie das Molkenproteinpulver auf die Aktivität des ACE zu untersuchen, wurden sowohl die Enzyme einzeln, in der Mischung in der sie auch eingesetzt wurden und eine Lösung des Milei60 ohne Enzymzugabe analog der Hydrolysate bei 100 °C für 10 Minuten hitzebehandelt. Dies geschah um die Proteasen zu denaturieren, da ansonsten auch ein Verdau des ACE und damit eine Hemmung dieses in Betracht zu ziehen ist. Die Untersuchungen ergaben, das weder die inaktivierten Enzyme, einzeln, wie auch als Mischung, noch das Protein einen Einfluss auf das ACE hatten. Dagegen zeigte eine Lösung des Molkenproteinpulvers und den drei Enzymen, welche sofort für 10 Minuten bei 100 °C zur Inaktivierung der Enzyme inkubiert wurde, eine verhältnismäßig starke ACE-Hemmung. Offenbar reichten wenige Minuten aus, eine gut messbare Hydrolyse der Molkenproteine zu verursachen und dabei ACE-hemmende Peptide freizusetzen. Dies belegt den starken enzymatischen Abbau, der durch die verwendeten Proteasen erreicht wird.

Im weiteren Verlauf der Studie zeigte jedes hergestellte Hydrolysat ein ACE-inhibierendes Potential. Für einen besseren Vergleich der Daten wurden die IC₅₀-Werte jeder Probe bestimmt und sind in Tab. 9 dargestellt.

**Tab. 9: Darstellung der IC₅₀-Werte des unterschiedlich lang hydrolysierten Molkenproteins**

| **Hydrolysezeit [h]** | **IC₅₀ [mg Protein/l]** | **Hydrolysezeit [h]** | **IC₅₀ [mg Protein/l]** |
|---|---|---|---|
| 0 | 180 | 4 | 42 |
| 0,17 | 176 | 8 | 90 |
| 0,5 | 97 | 12 | 74 |
| 1 | 96 | 24 | 36 |
| 2 | 104 | 30 | 36 |
| 3 | 90 | 48 | 76 |

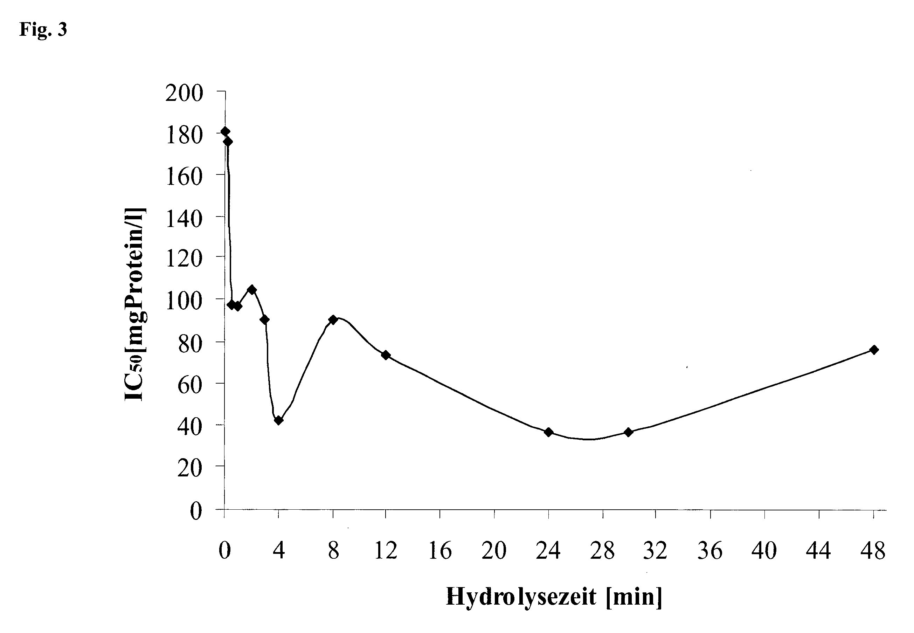

Das hemmende Potential der Hydrolysate nahm innerhalb der ersten vier Stunden stetig zu, bis für das 4 h-Hydrolysat ein sehr niedriger IC₅₀-Wert von nur 42 mg Protein/l ermittelt wurde. Danach nahm der hemmende Effekt zunächst wieder ab. Nach acht Stunden Hydrolyse ist mehr als doppelt soviel von der Probe nötig, um die gleichen Effekt wie das 4 h-Hydrolysat zu erzielen. Überraschenderweise nahm der hemmende Effekt dann wieder zu und erreichte nach 24 bzw. 30 die stärkste inhibierende Wirkung, welche allerdings nach 48 Stunden Hydrolysedauer wieder verringert wurde. Veranschaulicht werden diese Ergebnisse nochmals in **Figur 3****,** in der der IC₅₀-Wert der Hydrolysate gegen die Dauer der Hydrolyse aufgetragen ist.

Der oszillierende Verlauf des inhibierenden Potentials im Verlauf der Hydrolyse der Molkenproteine kann damit erklärt werden, dass es zur Bildung sowie auch zum Abbau ACE-hemmender Verbindungen kam. Da der proteolytische Abbau mit der Zeit immer langsamer wird, kann auch davon ausgegangen werden, dass auch die Veränderung des ACEhemmenden Potentials mit Zunahme der Hydrolysedauer immer langsamer voranschreitet. Überraschenderweise ruft das 24 h-Hydrolysat eine ebenso starke Reduzierung der ACE-Aktivität hervor wie das 4 h-Hydrolysat. Dabei ist davon auszugehen, dass diese Wirkung auf unterschiedlichen Inhibitoren beruht, denn mit voranschreitender Inkubationszeit werden die Molkenproteinkomponenten besser für die Enzyme zugänglich, so dass potente Inhibitoren wie beispielsweise das Ile-Trp (α-La 59-60) erst später freigesetzt werden können. Auch lagen in dem 24 h-Hydrolysat mehr Verbindungen mit einer molekularen Masse zwischen 1 und 0,175 kDa, als im 4 h-Hydrolysat vor.

### Ausführungsbeispiel 3:

Die Freisetzung der erfindungsgemäßen Dipeptide Trp-Leu und Ile-Trp im Verlauf der Hydrolyse wurde durch GPC und LC-ESI-TOF-MS (liquid chromatography/electrospray ionization time-of-flight mass spectrometry; Flüssigkeitschromatographie/Elektronenspray-Ionisation-Flugzeitmassenspektrometrie) analysiert.

Um die erfindungsgemäßen Dipeptide eindeutig identifizieren zu können, erfolgte zunächst eine Auftrennung eines Hydrolysats mittels RP-HPLC mit anschließender Detektion im UV bei 220 und 280 nm. Die Molmassen der einzelnen Peaks wurden mit einem hochauflösenden ESI-TOF-Massenspektrometer ermittelt. Das Prinzip der Elektrospray-Ionisation (ESI) beruht darauf, dass die Probelösungen zu einem feinen Nebel versprüht werden, aus dem das Lösungsmittel vollständig verdampfen kann. Durch Ladungsübertragung enthalten die zurückbleibenden Probenmoleküle ein oder mehrere Protonen aus dem Lösungsmittel. Die Ionen werden anschließend im Flugzeitanalysator (time of flight = TOF) nach ihrem Masse/Ladungs-Verhältnis (m/z) aufgetrennt. Aus den ermittelten Molmassen können schließlich mit dem Programm Data Explorer^{™} die möglichen Aminosäurezusammensetzungen bestimmt werden.

Für diese Analysenmethode wurden ausgewählte Hydrolysate in einer Konzentration von 40 mg/ml eingesetzt und membranfiltriert. Die verwendeten HPLC-Parameter sind in Tab. 10 aufgelistet.

**Tab. 10: HPLC-Parameter für LC-ESI-TOF-MS**

| | |
|---|---|
| Säule: | Eurospher-100 C 18; 250 x 4,6 mm, Packungsdurchmesser 5 µm |
| Einspritzvo lumen: | 50 µl |
| Säulentemperatur: | 35 °C |
| Flussrate: | 0,7 ml / min |
| Detektion: | UV (λ = 220 und 280 nm) und MS |

| Elutionssystem: | |
|---|---|
| Eluent A: | Acetonitril/ 5 mM Ammoniumacetatpuffer (pH 5,5) 84 + 16 (v/v) |
| | 840 ml Acetonitril (HPLC grade) mit 160 ml Fließmittel B mischen, membranfiltrieren und entgasen |
| Eluent B: | 5 mM Ammoniumacetatpuffer (pH 5,5) |
| | 0,385 g CH₃COONH₄ in 800 ml Reinstwasser lösen, pH-Wert der Lösung mit 3 %iger Essigsäure auf 5,5 einstellen, mit Reinstwasser auf 1 1 auffüllen, membranfiltrieren |

Die analysierten Massen wurden mit den mitgeführten Standardpeptiden Ile-Trp und Trp-Leu (Bachem Distribution Services GmbH, Weil am Rhein, Deutschland) verglichen. Das Einspritzvolumen der Probe betrug 50 µl. Das Gradientensystem ist in Tabelle 11 dargestellt.

**Tab. 11: Parameter der Gradientenelution der LC-ESI-TOF-MS-Untersuchungen**

| Zeit [min] | Eluent A [%] |
|---|---|
| 0 | 5 |
| 10 | 5 |
| 14 | 8 |
| 23 | 45 |
| 29 | 69 |
| 44 | 80 |
| 48 | 80 |
| 50 | 5 |
| 57 | 5 |

In Tab. 12 sind die für die erfindungsgemäßen Dipeptide per RP-HPLC und LC-ESI-TOF-MS gemessenen Parameter zusammengefasst. Die Tabelle enthält außerdem die für die Dipeptide erhaltenen IC₅₀-Werte. Die Bestimmung der enzymhemmenden Aktivität erfolgte wie in Ausführungsbeispiel 2 beschrieben, eingesetzt wurden dafür die Standardpeptide Ile-Trp und Trp-Leu (Bachem Distribution Services GmbH, Weil am Rhein, Deutschland).

In der GPC erfolgte die Identifizierung der Dipeptide über den Vergleich mit den Peaks der mitgeführten Standardpeptide.

**Tab. 12 Retentionszeiten (RP-HPLC), Peptidmassen und ACE-hemmende Aktivität der untersuchten Dipeptide**

| Retentions-zeit (RP-HPLC) [min] | Detektierte Peptidmasse M+H⁺ [Da] | Theoretische Peptidmasse M+H⁺ | Sequenz | IC₅₀ [µM] |
|---|---|---|---|---|
| **26,2** | **318,2** | **318,17** | **Ile-Trp** | **0,7 ± 0,3** |
| **27,3** | **318,2** | **318,17** | **Trp-Leu** | **10 ± 1,7** |

Das stark ACE-inhibierende Peptid **Ile-Trp** wurde eindeutig in den Hydrolysaten nach einer Hydrolysedauer von drei Stunden nachgewiesen. Es scheint also erst nach einer bestimmten Zeit für die Proteasen zugänglich zu sein, ebenso wie auch das **Trp-Leu.** Diese beiden Peptide schienen jedoch stabil gegen einen weiteren Verdau zu den Aminosäuren zu sein, denn durch GPC konnten die beiden Peptide in allen nachfolgenden Hydrolysaten bestimmt werden.

### Ausführungsbeispiel 4. Fütterungsversuche an spontan hypertensiven Ratten

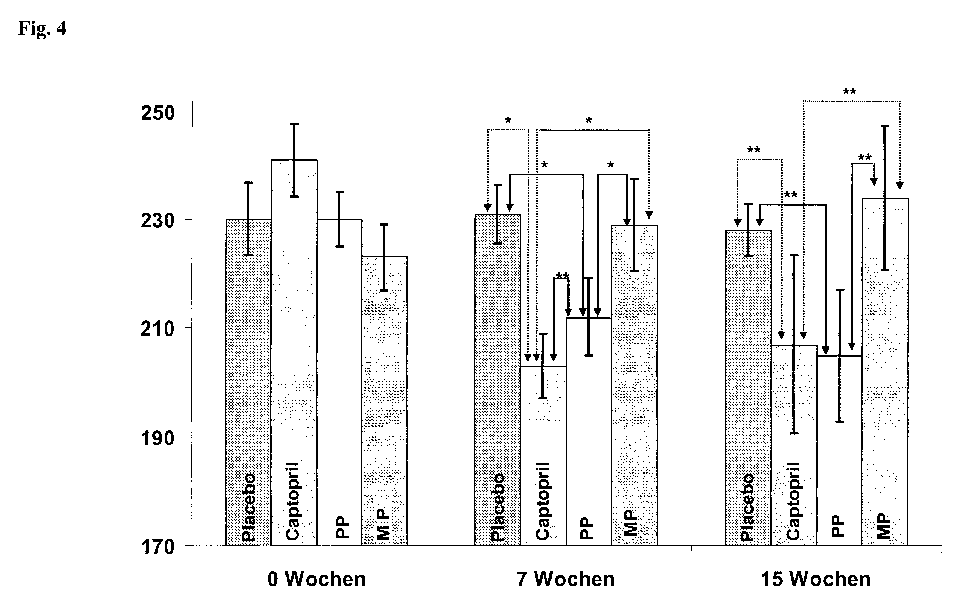

Die grundsätzliche Bedeutung von Molkenproteinhydrolysaten, die die erfindungsgemäßen tryptophanhaltigen Peptide enthielten, für die Beeinflussung des arteriellen Blutdrucks wurde im Tierversuch an spontan hypertensiven Ratten objektiviert (Figur 4). Hier führte das Molkenproteinhydrolysat im Vergleich zu einer Kontrolldiät zu einer signifikanten Senkung des systolischen Blutdrucks um 21 ± 6 mmHg nach 7 Wochen. Die Fütterung von Captopril (ACE-Hemmer der ersten Generation) senkte im gleichen Zeitraum den Blutdruck um 28 ± 7 mmHg.

Weitere günstige Effekte der Molkenproteinhydrolysate war nach Verfütterung des Präparates mit den erfindungsgemäßen tryptophanhaltigen Peptiden eine im Vergleich zur Kontrolle um 8 % geringere Herzmasse (Captopril: 16 % geringer), und die koronare Flussreserve war um 75 % im Vergleich zur Kontrolle gesteigert. Dies legt umfassende herz- und gefäßprotektive Effekte ausgelöst durch das Molkenproteinhydrolysat nahe.

## Patentansprüche

1. Molkenproteinhydrolysat erhältlich durch Hydrolyse von Molke, handelsübliches Molkepulver, Molkenproteinisolat, Molkenproteinpräparate oder aus Molke gewonnenem α-Lactalbumin
- mit wenigstens den Enzymen Alcalase und Trypsin mit einem Enzym/Substrat-Verhältnis, g Enzym /g Substrat, zwischen 1:10 und 1:10000,
- wobei die enzymatische Hydrolyse bei einer Temperatur bei einer Temperatur zwischen 30 °C und 70 °C und einer Inkubationszeit von 18 h bis 30 h erfolgt,
- bis mehr als 50 % des enthaltenen Proteins eine Molmasse von kleiner 4 kDa, bevorzugt kleiner 1 kDa, besonders bevorzugt kleiner 500 Da, besitzen,
- wobei die Hydrolyse nach Erreichen des gewünschten Hydrolysegrads durch Inaktivierung der Enzyme durch Erhitzen auf eine Temperatur vom 80 °C bis 100 °C abgestoppt wird.

2. Molkenproteinhydrolysat nach Anspruch 1, **dadurch gekennzeichnet, dass** der Hydrolyseschritt bevorzugt 20 h bis 28 h dauert.

3. Molkenproteinhydrolysat nach Anspruch 1 oder 2, **dadurch gekennzeichnet, dass** die enzymatische Hydrolyse bei einer Temperatur zwischen 50 °C und 60 °C durchgeführt wird.

4. Molkenproteinhydrolysat nach Anspruch 1 bis 3, **dadurch gekennzeichnet, dass** der Verdau mit den beiden Enzymen aufeinander folgt oder gleichzeitig durchführt wird.

5. Molkenproteinhydrolysat nach Anspruch 1 bis 4, **dadurch gekennzeichnet, dass** Verdaue mit weiteren Enzymen angeschlossen, oder weitere Enzyme dem Verdau mit Alcalase und Trypsin zugesetzt werden, wobei es sich bei den verwendeten Enzymen um Endoproteasen handelt.

6. Molkenproteinhydrolysat nach Anspruch 5, **dadurch gekennzeichnet, dass**, es sich um die Enzyme Chymotrypsin, Pancreatin, Pepsin und CorolasePP handelt.

7. Verfahren zur Herstellung eines Molkenproteinhydrolysats mit den Schritten:
- Hydrolyse von Molke, handelsübliches Molkenpulver, Molkenproteinisolat, Molkenproteinpräparate oder aus Molke gewonnenem α-Lactalbumin mit wenigstens, den Enzymen Alcalase und Trypsin mit einem Enzym/Substrat-Verhältnis, g/Enzym/g/Substrat zwischen 1:10 und 1:10.000, wobei die enzymatische Hydrolyse bei einer Temperatur zwischen 30 °C und 70 °C und einer Inkubationszeit von 18 h bis 30 h erfolgt bis mehr als 50 % des enthaltenen Proteins eine Molmasse von kleiner 4 kDa, bevorzugt kleiner 1 kDa, besonders bevorzugt kleiner 500 Da, besitzen,
- Abstoppen der Hydrolyse nach Erreichen des gewünschten Hydrolysegrads durch Inaktivierung der Enzyme durch Erhitzen auf eine Temperatur vom 80 °C bis 100 °C.

8. Verfahren nach Anspruch 7, **dadurch gekennzeichnet, dass** der Hydrolyseschritt bevorzugt 20 h bis 28 h dauert.

9. Verfahren nach Anspruch 7 oder 8, **dadurch gekennzeichnet, dass** die enzymatische Hydrolyse bei einer Temperatur zwischen 50 °C und 60 °C durchgeführt wird.

10. Verfahren nach Anspruch 7 bis 9, **dadurch gekennzeichnet, dass** der Verdau mit den beiden Enzymen aufeinander folgt oder gleichzeitig durchführt wird.

11. Verfahren nach Anspruch 7 bis 10, **dadurch gekennzeichnet, dass** Verdaue mit weiteren Enzymen angeschlossen, oder weitere Enzyme dem Verdau mit Alcalase und Trypsin zugesetzt werden, wobei es sich bei den verwendeten Enzymen um Endoproteasen handelt.

12. Verfahren nach Anspruch 11, **dadurch gekennzeichnet, dass**, es sich um die Enzyme Chymotrypsin, Pancreatin, Pepsin und CorolasePP handelt.

13. Verwendung eines Molkenproteinhydrolysat nach einem der Ansprüche 1 bis 6 zur Herstellung eines Arzneimittels, eines Nahrungsprodukts, eines Tierarzneimittels oder eines Futtermittels.

14. Arzneimittel, enthaltend ein Molkenproteinhydrolysat nach einem der Ansprüche 1 bis 6.

15. Nahrungsprodukt, enthaltend ein Molkenproteinhydrolysat nach einem der Ansprüche 1 bis 6.

16. Futtermittel, enthaltend ein Molkenproteinhydrolysat nach einem der Ansprüche 1 bis 6.

## Claims

1. Whey protein hydrolysate, obtainable by whey hydrolysis, commercially available whey powder, whey protein isolate, whey protein preparations or by α-Lactalbumin obtained from whey
- containing at least the enzymes alcalase and trypsin, and having an enzyme/substrate ratio of g enzyme / g substrate between 1:10 and 1: 10000,
- whereby the enzymatic hydrolysis takes place at a temperature of between 30 °C and 70 °C and within an incubation period of between 18 hours and 30 hours
- until more than 50 % of the protein contained have a molecular weight of less than 4 kDa, preferably less than 1 kDa, particularly preferably less than 500 Da,
- and whereby the hydrolysis is stopped after achieving the desired hydrolysis degree by inactivating the enzymes by means of heating them to a temperature of 80 °C to 100 °C.

2. Whey protein hydrolysate according to claim 1, **characterized in that** the hydrolysis step preferably takes 20 hours to 28 hours.

3. Whey protein hydrolysate according to claim 1 or 2, **characterized in that** the enzymatic hydrolysis takes place at a temperature of between 50 °C and 60 °C.

4. Whey protein hydrolysate according to claims 1 to 3, **characterized in that** the digestion with the two enzymes either follows one after the other, or takes place simultaneously.

5. Whey protein hydrolysate according to claims 1 to 4, **characterized in that** digestions with further enzymes take place subsequently, or **in that** further enzymes are added to the digestion with alcalase and trypsin, whereby the enzymes used are endoproteases.

6. Whey protein hydrolysate according to claim 5, **characterized in that** the enzymes used are chymotrypsin, pancreatin, pepsin and corolasePP.

7. Method for producing a whey protein hydrolysate comprising the steps:
- hydrolysis of whey, commercially available whey powder, whey protein isolate, whey protein preparations or α-Lactalbumin obtained from whey with at least the enzymes alcalase and trypsin, and having an enzyme/substrate ratio of g enzyme / g substrate between 1:10 and 1: 10,000, whereby the enzymatic hydrolysis takes place at a temperature of between 30 °C and 70 °C and within an incubation period of between 18 hours and 30 hours, until more than 50 % of the protein contained have a molecular weight of less than 4 kDa, preferably less than 1 kDa, particularly preferably less than 500 Da,
- Stopping the hydrolysis after achieving the desired hydrolysis degree by inactivating the enzymes by means of heating them to a temperature of 80 °C to 100 °C.

8. Method according to claim 7, **characterized in that** the hydrolysis step preferably takes 20 hours to 28 hours.

9. Method according to claim 7 or 8, **characterized in that** the enzymatic hydrolysis takes place at a temperature of between 50 °C and 60 °C.

10. Method according to claims 7 to 9, **characterized in that** the digestion with the two enzymes either follows one after the other, or takes place simultaneously.

11. Method according to claims 7 to 10, **characterized in that** digestions with further enzymes take place subsequently, or **in that** further enzymes are added to the digestion with alcalase and trypsin, whereby the enzymes used are endoproteases.

12. Method according to claim 11, **characterized in that** the enzymes used are chymotrypsin, pancreatin, pepsin and corolasePP.

13. Use of a whey protein hydrolysate according to any of the claims 1 to 6, for producing a medicament, a food product, a veterinarian medicament, or animal feed.

14. Medicament, containing a whey protein hydrolysate according to any of the claims1 to 6.

15. Food product, containing a whey protein hydrolysate according to any of the claims 1 to 6.

16. Animal feed, containing a whey protein hydrolysate according to any of the claims1 to 6.

## Revendications

1. Hydrolysat de protéine de lactosérum pouvant être obtenu par hydrolyse de lactosérum, de poudre de lactosérum du commerce, d'isolat protéique de lactosérum, de préparations protéiques de lactosérum ou d'α-lactalbumine obtenue à partir de lactosérum,
- comportant au moins les enzymes alcalase et trypsine avec un rapport enzyme-substrat, en g d'enzyme/g de substrat, compris entre 1:10 et 1:10 000 ;
- où l'hydrolyse enzymatique est réalisée à une température comprise entre 30 °C et 70 °C et sur une durée d'incubation de 18 heures à 30 heures ;
- jusqu'à plus de 50 % de la protéine obtenue possédant une masse molaire inférieure à 4 kDa, de préférence inférieure à 1 kDA, de façon particulièrement préférée inférieure à 500 Da ;
- où l'hydrolyse, après avoir atteint le degré d'hydrolyse souhaité, est arrêtée par inactivation des enzymes par chauffage à une température comprise entre 80 °C et 100 °C.

2. Hydrolysat de protéine de lactosérum selon la revendication 1, **caractérisé en ce que** l'étape d'hydrolyse dure de préférence 20 h à 28 h.

3. Hydrolysat de protéine de lactosérum selon la revendication 1 ou 2, **caractérisé en ce que** l'hydrolyse enzymatique est conduite à une température comprise entre 50 °C et 60 °C.

4. Hydrolysat de protéine de lactosérum selon la revendication 1 à 3, **caractérisé en ce que** la digestion avec les deux enzymes est réalisée consécutivement ou simultanément.

5. Hydrolysat de protéine de lactosérum selon la revendication 1 à 4, **caractérisé en ce que** suivent des digestions avec d'autres enzymes, ou **en ce que** d'autres enzymes sont ajoutées à la digestion avec l'alcalase et la trypsine, et il s'agit alors concernant les enzymes utilisées d'endoprotéases.

6. Hydrolysat de protéine de lactosérum selon la revendication 5, **caractérisé en ce qu'**il s'agit des enzymes chymotrypsine, pancréatine, pepsine et Corolase PP.

7. Procédé de fabrication d'un hydrolysat de protéine de lactosérum, comportant les étapes suivantes :
- hydrolyse de lactosérum, de poudre de lactosérum du commerce, d'isolat protéique de lactosérum, de préparations protéiques de lactosérum ou d'α-lactalbumine obtenue à partir de lactosérum avec au moins les enzymes alcalase et trypsine selon un rapport enzyme/substrat, en g d'enzyme/g de substrat, compris entre 1:10 et 1:10 000, où l'hydrolyse enzymatique est réalisée à une température comprise entre 30 °C et 70 °C et sur une durée d'incubation comprise entre 18 h et 30 h, jusqu'à plus de 50 % de la protéine obtenue possédant une masse molaire inférieure à 4 kDa, de préférence inférieure à 1 kDa, de façon particulièrement préférée inférieure à 500 Da,
- arrêt de l'hydrolyse après avoir atteint le degré d'hydrolyse souhaité, par inactivation des enzymes par chauffage à une température comprise entre 80 °C et 100 °C.

8. Procédé selon la revendication 7, **caractérisé en ce que** l'étape d'hydrolyse dure de préférence 20 h à 28 h.

9. Procédé selon la revendication 7 ou 8, **caractérisé en ce que** l'hydrolyse enzymatique est réalisée à une température comprise entre 50 °C et 60 °C.

10. Procédé selon la revendication 7 à 9, **caractérisé en ce que** la digestion avec les deux enzymes est réalisée consécutivement ou simultanément.

11. Procédé selon la revendication 7 à 10, **caractérisé en ce que** suivent des digestions avec d'autres enzymes, ou **en ce que** d'autres enzymes sont ajoutées à la digestion avec l'alcalase et la trypsine, et il s'agit alors concernant les enzymes utilisées d'endoprotéases.

12. Procédé selon la revendication 11, **caractérisé en ce qu'**il s'agit des enzymes chymotrypsine, pancréatine, pepsine et Corolase PP.

13. Utilisation d'un hydrolysat de protéine de lactosérum selon l'une des revendications 1 à 6 pour fabriquer un médicament, un produit alimentaire, un médicament vétérinaire ou un aliment pour animaux.

14. Médicament contenant un hydrolysat de protéine de lactosérum selon l'une des revendications 1 à 6.

15. Produit alimentaire contenant un hydrolysat de protéine de lactosérum selon l'une des revendications 1 à 6.

16. Aliment pour animaux contenant un hydrolysat de protéine de lactosérum selon l'une des revendications 1 à 6.
